# EUROPEAN PATENT SPECIFICATION

(11) **EP 1 550 462 B1**
(45) Date of publication and mention of the grant of the patent: **25.01.2012**
(21) Application number: 03792648.2
(22) Date of filing: 05.08.2003
(51) Int. Cl.: A61K 45/00, A61K 31/122, A61K 31/352, A61K 31/7056, A61K 31/711, A61K 31/4439, A61K 31/497, A61K 31/4709, A61K 31/4196, A61K 31/4184, A61K 31/336, A61P 13/12

(54) **REMEDY OR PREVENTIVE FOR KIDNEY DISEASE AND METHOD OF DIAGNOSING KIDNEY DISEASE**
MITTEL ZUR BEHANDLUNG ODER PRÄVENTION VON NIERENERKRANKUNGEN UND VERFAHREN ZUR DIAGNOSE VON NIERENERKRANKUNGEN
REMEDE OU AGENT PREVENTIF CONTRE UNE MALADIE DES REINS ET PROCEDE DE DIAGNOSTIC D'UNE MALADIE DES REINS

(30) Priority: 06.08.2002 JP 2002229262
(43) Date of publication of application: 06.07.2005
(73) Proprietor: TORAY INDUSTRIES, INC., Tokyo 103-8666 (JP)
(72) Inventor: YAMADA, Masateru, Kamakura-shi, Kanagawa 248-0034 (JP); KURUMATANI, Hajimu, Fujisawa-shi, Kanagawa 251-0038 (JP); SUDO, Tetsuo, Fujisawa-shi, Kanagawa 251-0027 (JP); TSUJIMOTO, Gozoh, Tokyo, 158-0091 (JP)
(74) Representative: Hofer, Dorothea
(86) International application number: PCT/JP2003/009910
(87) International publication number: WO 2004/017997

(56) References cited:
- EP-A1- 0 956 867
- EP-A1- 0 997 146
- WO-A1-01/00610
- WO-A1-01/70248
- WO-A1-02/057240
- WO-A2-01/42231
- WO-A2-02/24681
- WO-A2-2004/058185
- JP-A- 2000 080 035
- JP-A- 2002 220 334
- US-B1- 6 274 611
- LIN J -K ET AL: "Antiinflammatory and antitumor effects of flavonoids and flavanoids" DRUGS OF THE FUTURE, vol. 26, no. 2, 2001, pages 145-152, XP002554889 ES ISSN: 0377-8282
- SARNO STEFANIA ET AL: "Selectivity of 4,5,6,7-tetrabromobenzotriazole, an ATP site-directed inhibitor of protein kinase CK2 ('casein kinase-2')" FEBS LETTERS, vol. 496, no. 1, 4 May 2001 (2001-05-04), pages 44-48, XP004239417 ISSN: 0014-5793
- SARNO STEFANIA ET AL: "Toward the rational design of protein kinase casein kinase-2 inhibitors." PHARMACOLOGY & THERAPEUTICS 2002 FEB-MAR, vol. 93, no. 2-3, February 2002 (2002-02), pages 159-168, XP002554891 ISSN: 0163-7258
- NEGULESCU OLIVIA ET AL: "Estradiol reverses TGF-beta1-induced mesangial cell apoptosis by a casein kinase 2-dependent mechanism." KIDNEY INTERNATIONAL, vol. 62, no. 6, December 2002 (2002-12), pages 1989-1998, XP002554892 ISSN: 0085-2538
- YOKOZAWA TAKAKO ET AL: "Protective effects of some flavonoids on the renal cellular membrane" EXPERIMENTAL AND TOXICOLOGIC PATHOLOGY, vol. 51, no. 1, January 1999 (1999-01), pages 9-14, XP009125581 ISSN: 0940-2993
- DATABASE WPI Week 200224 Thomson Scientific, London, GB; AN 1999-395084 XP002554898 & CN 1 324 612 A (INST NEPHROPATHY PLA) 5 December 2001 (2001-12-05)
- KUO YUH-CHI ET AL: "Immune responses in human mesangial cells regulated by emodin from Polygonum hypoleucum Ohwi" LIFE SCIENCES, vol. 68, no. 11, 2 February 2001 (2001-02-02), pages 1271-1286, XP002554894 ISSN: 0024-3205
- YAMADA MASATERU ET AL: "Inhibition of protein kinase CK2 prevents the progression of glomerulonephritis" PROCEEDINGS OF THE NATIONAL ACADEMY OF SCIENCES OF THE UNITED STATES OF AMERICA, vol. 102, no. 21, May 2005 (2005-05), pages 7736-7741, XP002554895 ISSN: 0027-8424
- NASSAR GEORGE M ET AL: "Novel approaches to treatment of glomerulonephritis" JN JOURNAL OF NEPHROLOGY, vol. 11, no. 4, July 1998 (1998-07), pages 177-184, XP009125506 ISSN: 1121-8428
- ANDERSON R J: "Recent advances and developments in the treatment of acute renal failure" EXPERT OPINION ON THERAPEUTIC PATENTS, vol. 12, no. 5, May 2002 (2002-05), pages 645-655, XP002554897 GB ISSN: 1354-3776
- MAKINO TOSHIAKI ET AL.: 'Inhibitory effect of decoction of perilla fruttescens on cultured murine mesangial cell proliferation and quantitative analysis of its active constituents' PLANTA MEDICA vol. 67, no. 1, 2000, pages 24 - 28, XP002976534
- WANG HUAMIN ET AL.: 'Response of cancer cells to molecular interruption of the CK2 signal' MOLECULAR AND CELLULAR BIOCHEMISTRY vol. 227, no. 1-2, 2001, pages 167 - 174, XP009027790
- DANIOTTI JOSE L. ET AL.: 'Cloning and expression of genes coding for protein kinase CK2 <SYM97> and <SYM98> subunits in zebrafish (Danio rerio)' CELLULAR & MOLECULAR BIOLOGY RESEARCH vol. 40, no. 5/6, 1994, pages 431 - 439, XP002976535
- PEPPERKOK R. ET AL.: 'Cell growth stimulation by EGF: inhibition through antisense-oligodeoxynucleotides demonstrates important role of casein kinase II' EXPERIMENTAL CELL RESEARCH vol. 197, no. 2, 1991, pages 245 - 253, XP002952117

## Description

### Technical Field

The present invention relates to an agent for use in therapy and/or prevention of kidney diseases, and to a method for diagnosis of kidney diseases.

### Technical Background

Kidney is a paired urinary organ located at backside in body cavity, which serve to keep homeostasis through 1) excretion of water, 2) excretion of metabolites, especially nitrogen components, 3) excretion of electrolytes, 4) excretion of foreign matters and 5) adjustment of blood osmosis pressure, amount of body fluid and acid-base equilibrium, by generation of urine. Kidney is an organ having important physiological functions such as adjustment of blood pressure through production and secretion of renin and prostaglandins, adjustment of differentiation and maturation of erythrocytes in bone marrow through production of erythropoietin, and activation of 25-hydroxyvitamin D which is a precursor of vitamin D (Blood, 68 (Suppl), 170a, 1986, Proc Natl Acad Sci USA, 28, 1199, 1981). Kidney is an aggregate of about 1,000,000 functional units called nephron, and nephron comprises glomerulus, Bowman's capsule, proximal renal tubule, Henle's loop and distal renal tubule. Nephrons join to a collecting duct, and the collecting duct opens at renal pelvis. Glomerulus filters blood through a spherical agglomerate of blood capillaries to produce primitive urine. Primitive urine is subjected to reabsorption and secretion in renal tubules, thereby producing the final urine. Usually, glomeruli have controls such that necessary substances in the blood, especially serum proteins, do not leak during the filtration step. However, when the glomeruli are disordered, proliferation of mesangial cells, a kind of the constituting cells, and increase of the peripheral matrix occur, so that the amount of proteins excreted to the urine is increased. Upon increase of the amount of the excreted proteins in the urine; the proteins *per se* damage the renal tubules, which exacerbates the damage of the glomeruli. This vicious circle rapidly decreases the renal function.

Representative kidney diseases include glomerular and tubulointerstitial nephritis, and diabetic nephropathy. Acute nephritic syndrome, rapidly progressive glomerulonephritic syndrome, recurrent or sustained hematuria syndrome, chronic nephritic syndrome, diabetic nephropathy, lupus nephritis, IgA nephropathy, chronic pyelitis, nephrosclerosis, renal hypertension, gouty kidney, acute or chronic renal failure and so on are known. Although kidney diseases present complicated and a variety of pathoses, any of them may follow severe course reaching renal failure. Known therapeutic uses for kidney diseases performed depending on the severity include therapies for retaining renal functions, such as rest cure, diet cure and pharmacotherapy; hemodialysis therapy and kidney transplantation. Upon reaching renal failure, dialysis therapy such as hemodialysis or peritoneal dialysis, or kidney transplantation is necessary. Although the hemodialysis therapy is said to be the ultimate therapy of kidney diseases, it merely eliminates wastes accumulated in the body due to the renal function disorder, and the function of severely damaged kidney is not recovered, so that the patient has to continue the dialysis therapy accompanying pain and troubles, which requires equipment and costs, throughout the life. Further, eventually, in most cases, heart failure, an infectious disease or the like is involved to death. Recently, the number of patients who start dialysis is increasing year by year, which is problematic from the viewpoint of economy and society. Although the only radical treatment of renal failure is kidney transplantation, since it has a number of problems including shortage of donors, difficulties in tissue compatibility and avoidance of rejection reaction, the number of cases is small. Thus, development of radical therapy or radical therapeutic agent is demanded. Although there are a number of diseases and pathoses which cause renal failure, the most frequent causative diseases are nephritis and diabetic nephropathy. Therefore, to provide therapy and prevention of these kidney diseases is one of the most important tasks in the field of kidney diseases. Nephritis includes glomerulonephritis starting from damage of glomeruli, and tubular nephritis starting from renal tubules. In the former case, deterioration of renal tubules occurs following the damage of glomeruli, while in the latter, the deterioration is limited to the renal tubules. Clinically important lupus nephritis, IgA nephritis and the like are included in glomerulonephritis, and its mechanism of onset is thought to include immunological mechanism and non-immunological mechanism. As an example of the former, deposition of an immune complex between an antigen originated from a bacterium such as staphylococcus or hemolytic streptococcus, or a virus such as hepatitis B virus or measles, and an antibody thereto, is known. However, it is thought today that there are a variety of causes, and there are many unsolved problems. By the retard of excretion of wastes in the body fluid due to sustained chronic nephritis, the burden to the kidney is increased, which further exacerbates the progress of nephritis, thereby progressing the deterioration. The kidney in which the glomeruli and renal tubules arc completely deteriorated to reach renal failure cannot recover by itself, and therapeutic use or therapeutic drug which may recover the kidney has not yet been completed. Although steroidal anti-intlammatory drugs are used against acute nephritis and light chronic nephritis, there are no radical therapeutic uses against renal failure reached after aggravation of chronic nephritis, and there is no way other than to continue dialysis therapy. However, the function of the kidney to secrete hormones and the like is also very important. Especially, if the amount of erythropoietin produced at proximal renal tubules is decreased due to the deterioration of the proximal renal tubules, severe anemia occurs. Therefore, accurate diagnosis and therapy at an early stage are demanded.

At present, for pharmacotherapy against nephritis, mainly steroidal anti-inflammatory drugs, as well as immunosuppressants, anti-platelet agents, antihypertensive agents, diuretic drugs and the like are used depending on the cause or pathosis. For diabetic nephropathy, together with strict glycemic control, antihypertensive agents such as angiotensin converting enzyme inhibitors, ATII receptor blockers and calcium blockers are used. However, with these known therapeutic agents against kidney diseases, it is difficult to sufficiently stop the progress to renal failure, and the present state is far from the radical therapy (Hiroshi OKA and Osamu WADA responsible eds., "Medical Science Unabridgcd Dictionary", Supplement 5, Latest Information of Therapies, p.218, Kodansha, 1988). Thus, satisfactory therapeutic agent against kidney diseases does not exist Further, side effects accompanied by continuous long term use of the drugs are also problematic. Thus, at present, therapy and prevention of kidney diseases are basically carried out by rest cure and/or diet cure. Pharmacotherapy against kidney diseases is still in the process of trial and error, and development of an effective drug is demanded.

### Disclosure of the Invention

Accordingly, an object of the present invention is to provide an effective agent for use in therapy and/or prevention of kidney diseases, and diagnostic method of kidney diseases.

For solving the above-described problems, the present inventors intensively studied using the nephritis of rat models induced by antibody to glomerular basement membrane. That is, the present inventors searched genes expressing in diseased kidney by comparing gene expression in the diseased kidney and in the non-diseased, kidney, to discover that casein kinase 2 is prominently increased in the diseased kidney. Further, the present inventors experimentally demonstrated that kidney diseases may be diagnosed using the expression of casein kinase 2 as an index, and that therapy and/or prevention of kidney diseases may be attained by administration of nucleic acid molecule which inhibits expression of casein kinase 2, thereby completing the present invention.

That is, the present invention provides an agent for use in therapy and/or prevention of kidney diseases, comprising as an effective ingredient a nucleic acid molecule which inhibits expression of casein Kinase 2. The present invention also provides a method for diagnosis of kidney diseases, comprising measuring activity and/or content of casein kinase 2, and/or measuring expression amount of casein kinase 2 gene in a sample separated from body. The present invention also provides use of the nucleic acid molecule which inhibits expression of casein kinase 2 for the production of an agent for use in therapy and/or prevention of kidney diseases.

The agent for use in therapy and/or prevention of kidney diseases according to the present invention is an excellent agent for therapy and/or prevention of kidney diseases for which sufficient effects are not expected by the known drugs. Further, by the diagnostic method of kidney diseases (detection method of kidney diseases) according to the present invention, pathoses of kidney diseases may be accurately and efficiently diagnosed or detected.

### BRIEF DESCRIPTION OF THE DRAWINGS

Fig. 1 shows relationship between the expression amount of casein kinase 2 gene in kidney tissue, i.e., in the cells constituting the kidney, and the amount of excreted urinary protein, i.e., the state of kidney disease.
Fig. 2 shows the effect of administration of an antisense oligonucleotide against casein kinase 2 α subunit on the amount of excreted urinary protein in nephritis rat models. *: p<0.05

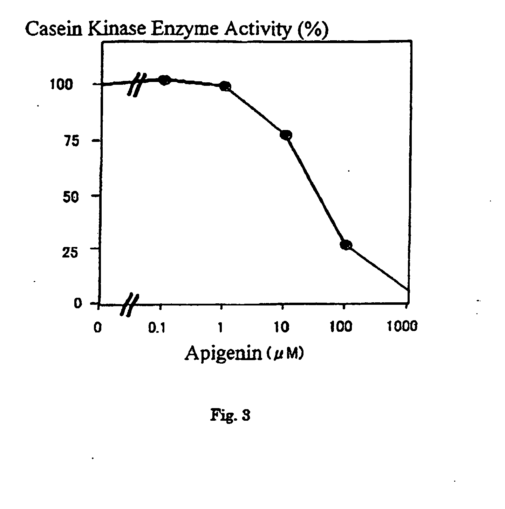

Fig. 3 shows inhibitory action of apigenin against enzyme activity of casein kinase 2.

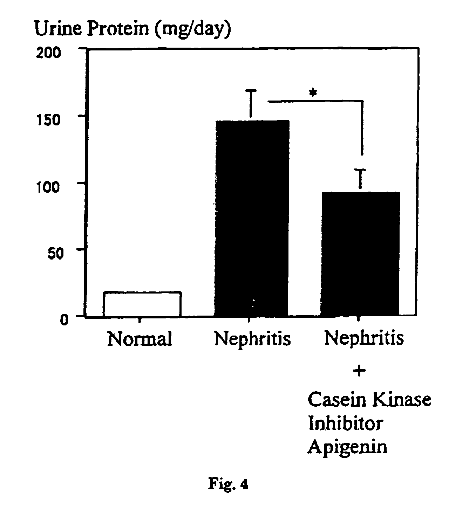

Fig. 4 shows the effect of administration of apigenin which is a casein kinase 2 inhibitor on the amount of excreted urinary protein in nephritis rat models. *: p<0.05

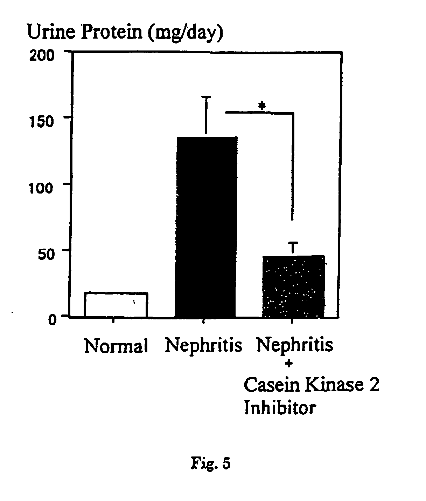

Fig. 5 shows the effect of administration of a casein kinase 2 inhibitor on the amount of excreted urinary protein in nephritis rat models. *: p<0.01

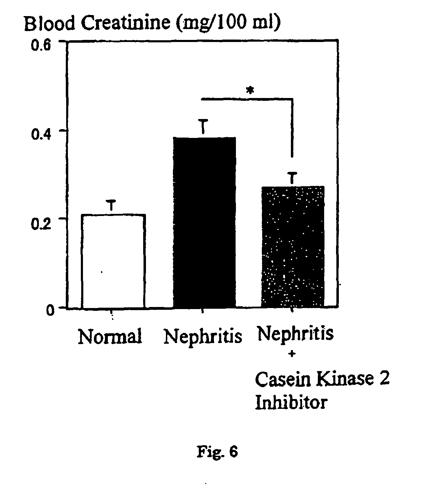

Fig. 6 shows the effect of administration of a casein kinase 2 inhibitor on the blood creatinine level in nephritis rat models. *: p<0.01

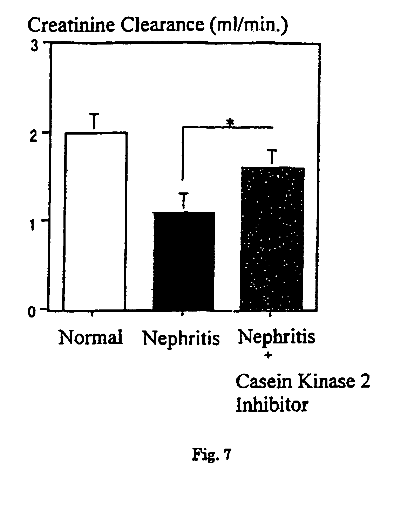

Fig. 7 shows the effect of administration of a casein kinase 2 inhibitor on the endogenous creatinine clearance in nephritis rat models. *: p<0.01

### BEST MODE FOR CARRYING OUT THE INVENTION

The kidney diseases to be treated and/or prevented by the agent for therapy and/or prevention of kidney diseases according to the present invention are not restricted, and examples thereof include glomerulonephritis, interstitial nephritis, diabetic nephropathy, and chronic and acute renal failure. Further examples include acute glomerulonephritis syndrome, acute progressive glomerulonephritis syndrome, recurrent or sustained hematuria syndrome, IgA nephropathy, chronic pyelitis, nepbrotic syndrome, lupus nephritis, renal hypertension, gouty kidney, acute tubulointerstitial nephritis, chronic tubulointerstitial nephritis, renal infarction, drug-induced nephropathy, dysfunction of transplanted kidney, renal function disorder accompanied by renal calculus or ureteral obstruction. Among these diseases, nondiabetic kidney diseases, *inter alia,* nephritis, are preferred. Especially, the above-described diseases characterized by increase of expression of casein kinase 2 and/or increase of enzyme activity are preferred. The present invention may be applied not only to human, but also to mammalians such as simian, canine feline, swine, bovine, ovine, goat, rabbit, rat and mouse. Thus, the agent for use according to the present invention may be used not only as a human drug, but also as an animal drug. The agent for use in therapy and/or prevention of kidney diseases is useful for therapy of kidney diseases, prevention for kidney diseases, and for simultaneous therapy of kidney diseases and prevention of exacerbation of diseased state.

As mentioned above, the agent for use in therapy and/or prevention of kidney diseases according to the present invention comprises as an effective ingredient a nucleic acid molecule which inhibits expression of casein kinase 2. Casein kinase 2 is a protein, and a kind of multifunctional protein kinase having a wide range of substrate specificity. It widely occurs in eukaryotic cells and exists in cytoplasms and nuclei. It consists of a subunit and α' subunit having a molecular weight of 41 - 44 kD, and β subunit having a molecular weight of 24 - 28 kD, and exists in the form of ααββ tetramer or αα'ββ tetramer having a molecular weight of 130-140 kD (Biochemistry, 28, 4072-4076(1989); Biochemistry, 29, 8436-8447(1990); Biochemistry, 28, 9053-9058(1989)). The active center resides in the α subunit and α' subunit, and the enzyme is thought to phosphorylate serine and threonine residues using ATP and GTP as the source of phosphate. However, up to now, the significance of casein kinase 2 in the body and the significance in the diseased state have not been well understood. Further, it was not known that casein kinase 2 is expressed in diseased kidney, and that kidney diseases may be cured and/or prevented by administering a substance which inhibits expression of casein kinase 2. The fact that kidney diseases may be prevented and/or cured by inhibiting expression of casein kinase 2 is not at all expected from the prior art, and was first discovered by the present inventors.

As will be concretely described in Examples below, the present inventors discovered that the expression amount of casein kinase 2 gene in the kidney cells of diseased kidney was significantly larger than that in normal kidney cells, and that the diseased state of the kidney was significantly improved by administering a nucleic acid molecule which inhibits expression of casein kinase 2. These nucleic acid molecules are useful as agents for use in therapy and/or prevention of kidney diseases for which effective therapeutic method did not exist. As will be described in Examples, in the cells constituting the kidney in diseased state, the expression amount of the casein kinase 2 gene is significantly larger than in the cells constituting normal kidney. The larger the expression amount of the casein kinase 2 gene in the cells constituting the kidney, the worse the state of the kidney disease, and the closer the expression amount to the normal level, the closer the state of the kidney disease to the normal state. This is not restricted to the expression of the gene, but is also true for the expression of the protein. As will be described in Examples, the expression amount of the casein kinase 2 protein, that is, the content of the protein, in the cells constituting diseased kidney is significantly larger than that in the cells constituting normal kidney. The larger the expression amount, i.e., the content, of the casein kinase 2 protein in the cells constituting the kidney, the worse the state of the kidney disease, and the closer the expression amount, i.e., the content, to the normal level, the closer the state of the kidney disease to the normal state. Further, the expression amount, i.e., the content, of the casein kinase 2 protein is very closely related to the enzyme activity, i.e., the function, of casein kinase 2. That is, as will be described in Examples, the larger the amount of the casein kinase 2 protein, the higher the enzyme activity, i.e., the function, of casein kinase 2. These facts indicate that the larger the expression amount of the casein kinase 2 gene, expression amount, i.e., the content, of the protein, and the enzyme activity, i.e., the function, the worse the state of the kidney disease, and the closer the amounts to the normal levels by suppression of the expression, the closer the state of the kidney disease to the normal state. Further, these facts give a generally recognizable theoretical ground that any means for decreasing or inhibiting the expression of the casein kinase 2 gene, expression amount. i.e., content of the protein, and/or enzyme activity, i.e., the function, as well as any substance which can decrease or inhibit these have actions or effects to make the state of the kidney disease close to the normal state, so that they have actions or effects for curing and preventing kidney diseases. For example, it is shown in Examples that means for inhibiting expression of casein kinase 2 by using an antisense oligonucleotide has an action or effect to make the state of the kidney disease close to the normal state, so that they have actions or effects for curing and preventing kidney diseases. In other words, it is shown in Examples that antisense oligonucleotides inhibiting expression of casein kinase 2, have an action or effect to make the state of the kidney disease close to the normal state, so that they have actions or effects for curing and preventing kidney diseases. That is, it is proved that, in general, means and substances having actions to inhibit expression of casein kinase 2 have actions to cure and prevent kidney diseases. Further, using the expression of the casein kinase 2 gene, the expression amount, i.e., the content, and the enzyme activity, i.e., the function, as an index, kidney diseases may be diagnosed. Casein kinase 2 is known, and those skilled in the art can conceive the means and substances which inhibit casein kinase 2, and these are also explained in detail in the present invention. Therefore, needless to say, any of these may be practiced by those skilled in the art within the scope of usual techniques.

These casein kinase 2-inhibiting nucleic acid molecules will now be described in more detail.

Examples of the above-described nucleic acid molecules which inhibit expression of casein kinase 2 gene include nucleic acids, which inhibit the expression (transcription, translation) of the gene or the protein of the subunit, of casein kinase 2. Preferred nucleic acids include antisense oligonucleotides, RNAis and ribozymes against the casein kinase 2 gene. Further, vectors for gene introduction for gene therapy, containing an antisense gene against casein kinase 2 gene, cells in which the antisense gene against casein kinase 2 gene was introduced by the vector, and agents for gene therapy containing as an effective ingredient the vectors for gene introduction for gene therapy or the cells in which the antisense gene against casein kinase 2 gene was introduced, are also induced in these examples.

In normal cells, genetic information is transferred through the flow of genetic DNA - mRNA - protein. The nucleotide sequence of the mRNA coding for the protein is called sense sequence, and the nucleotide sequence complementary to this sequence is called antisense sequence. Antisense sequences are explained as spontaneously generating biological suppressors against gene expression, found in both prokaryotes (T. Mizuno, M-Y Chou and M. Inoue, Proc. Natl. Acad. Sci. USA, Vol. 81, pp. 1966-1970, (1984)) and eukaryotes (S. M. Heywood , Nucleic Acids Res. Vol. 14, pp.6771-6772 (1986)). These sequences are thought to function to inhibit translation by being hybridized with the complementary mRNA (B. M. Paterson, B. E. Roberts and E. L. Kuff, Proc. Natl. Acad. Sci. USA, Vol. 74, pp.4370-4374, (1977). Oligonucleotides including antisense oligonucleotides specifically hybridize with the complementary nucleotide sequence of any of precursor mRNA and mature mRNA. This term includes naturally occurring oligomers consisting of bases, sugars and bonds between sugars (main chain), as well as oligomers having non-naturally occurring moieties, which function in the similar manner. In brief, antisense method is a method for nucleotide sequence-specifically inhibiting any of the expression of genetic DNA, function of mRNA or genetic DNA, translation to protein, transportation to cytoplasan, and optional activity necessary for the total biological functions by chemically synthesizing an antisense oligonucleotide having a nucleotide sequence complementary to mRNA transcribed from a genetic DNA, administering the antisense oligonucleotide to cells or individuel so as to make it specifically bind with the complementary single-or double stranded mRNA to form double-stranded structure. By this, since all or a part of the functions of the mRNA or the genetic DNA cannot be carried out, defect of a part of the genome regulating proper expression of the protein is resulted, and the flow of the genetic information from the genetic DNA to the protein is blocked. As a result, the expression of the gene is regulated in the negative direction and inhibited. Antisense method nucleotide sequence-specifically inhibits the expression of the gene having the same sequence as the antisense oligonucleotide. Many recent studies proved that the antisense oligonucleotides are useful for excellent methods for analyzing gene function (Rothentberg et al., J.Natl.Cancer Inst. 1989, 81, 1539-1544, Zon, G. Pharmaceutical Res. 1988, 5, 539-549).

Since an antisense oligonucleotide is a long chain composed of four types of base units, the antisense oligonucleotide against any target gene may easily be synthesized. By virtue of the recent progress of oligonucleotide chemistry, and by virtue of the recent progress in the syntheses of nuclease-resistant oligonucleotides, S-oligonucleotides and the like, use of antisense oligonucleotides as a novel therapeutic method can be devised now. Cells contain various types of exo- and endonucleases which can decompose nucleic acids. A number of modifications of nucleotides and nucleosides have been shown to give resistance to digestion by nucleases when compared with naturally occurring oligonucleotides. Such modified or substituted oligonucleotides are often preferred to those of the natural form. Nuclease resistance is routinely measured by incubating an oligonucleotide with a cell extract or isolated nuclease solution, and measuring the remaining intact oligonucleotide with time usually by gel electrophoresis. An oligonucleotide so modified as to promote the nuclease resistance remains intact for a longer time than the unmodified-oligonucleotide. Various modifications of oligonucleotides for promoting or giving nuclease resistance are known. Examples of the intended preferred modes of modification of oligonucleotide include those employing phosphorothioates as in S-oligonucleotides and those employing bonds between sugars through, phosphotriester, methyl phosphonate, short chain alkyl, cycloalkyl, short chain hetero atoms, or heterocyclic structure. S-oligonucleotide (nucleotide phosphorothioate) is an isoelectric analogue of an oligonucleotide (O-oligo) in which the oxygen atoms in the phosphate group, which oxygen atoms do not participate in the polycondensation, are substituted by sulfur atoms. S-oligonucleotide may be obtained by treating the corresponding O-oligo with 3H-1,2-benzodithiol-3-one-1,1-dioxide which is a sulfur-transferring reagent See R.P. Iyer et al., J. Org. Chem.. Vol. 55, pp. 4693-4698, (1990); and R.P. Iyer et al., J. Am. Chem. Soc. Vol. 112, pp. 1253-1254, (1990). The oligonucleotides having morpholino main chain structure are also preferred (Summerton, J.E. and Weller, D.D., U.S. Patent No. 5,034,506). In another preferred mode, the phosphodiester main chain of the oligonucleotide may be substituted by polyamide main chain such as protein-nucleic acid (PNA) main chain (P.E.Nielsen. M. Egholm, R. H. Berf, O. Buchardt, Science 1991,254,1497). In this case, each base is directly or indirectly bound to the aza nitrogen atom in the polyamide main chain. Oligonucleotide may have a sugar mimic such as cyclobutyl in place of pentofuranosyl group. Further, typically, the oligonucleotide has at least one modified nucleotide region which gives one or more advantageous properties (e.g., promotion of the nuclease resistance, increase in the uptake into cells, and increase in binding affinity to RNA target) and a region serving as a substrate for the cleavage by RNaseH. In a preferred mode, the oligonucleotide has at least one region modified for increasing the binding affinity to the target, and usually has a region serving as a substrate ofRNaseH. The affinity to the target (in this case, the nucleic acid coding for casein kinase 2) is routinely determined by measuring the melting temperature (Tm) of the oligonueleotide/target pair, which is the temperature at which the oligonucleotide and the target dissociate. The dissociation is detected spectrophotometrically. The higher the melting temperature (Tm), the higher the affinity of the oligonucleotide to the target. In a more preferred mode, the region in the oligonucleotide, which is modified for increasing the binding affinity to the mRNA of casein kinase 2 comprises an at least one nucleotide of which 2'-position of the sugar is modified (2'-O-alkyl- and 2'-fluoro-modified oligonucleotides are most preferred). Such a modification is routinely incorporated into the oligonucleotide, and the modified oligonucleotide exhibits higher melting temperature (Tm) (i.e., higher binding affinity to the target) than that of 2'-deoxyoligonucleotide. The resulting increased affinity very much promotes the effect of the antisense oligonucleotide against casein kinase 2. RNaseH is a cellular endonuclease which cleaves RNA chains in RNA:DNA duplexes. Therefore, activation of this enzyme results in the cleavage of the RNA target, and may very much promote the efficiency of the antisense inhibition. Cleavage of the RNA target may be routinely shown by gel electrophoresis.

Oligonucleotide or its analogue preferably contain about 12 to about 50, more preferably 12 to 25, still more preferably 16 to 22 bases. Further, such an oligonucleotide or its analogue specifically hybridizes with the target mRNA nucleotide sequence under physiological conditions (melting temperature (Tm) of substantially higher than 37°C, preferably at least 50°C, typically at 60°C to 80°C or higher). The hybridization preferably corresponds with the stringent hybridization conditions, and the conditions are so selected as to attain a temperature which is lower than the melting temperature (Tm) with the target mRNA nucleotide sequence at the ion strength and at the pH by about 10°C, preferably by about 5°C. Further, complementarity (the degree of complementariness of a polypeptide to another polypeptide) may be quantified by the ratio of bases expected to form hydrogen bonds each other according to the generally accepted base-paring rule. The terms "specifically hybridizable" and "complementary" are the terms used for describing the degree of complementarity which is sufficient to attain stable and specific binding between the target DNA or RNA and the oligonucleotide. It should be understood that 100% complementarity is not necessary to be able to specifically hybridize. However, 100% complementarity is most preferred for the specificity and good translation inhibition. Even in cases where the complementarity is not 100%, the complementarity is preferably not less than 90% (that is, the number of bases which mismatch with the bases of mRNA to be paired in the entire antisense oligonucleotide to be hybridized with the mRNA is not more than 10%). In cases where the binding of the oligonucleotide to the target interferes the normal function of the target molecule so as to eliminate the utility of the target molecule, and in cases where the oligonucleotide has a sufficient degree of complementarity to avoid non-specific binding of the oligonucleotide to the non-targeted sequence in the conditions under which the specific binding is desired, for example, under the physiological conditions in case of therapeutic treatment, the oligonucleotide is specifically hybridizable. Such an oligonucleotide or its analogue may be prepared simply and routinely by the well-known solid phase synthesis. As for the studies of chemical synthesis of oligonucleotides, see Goodchild, Bioconjugate Chemistry, Vol. 1, pp. 165-167, (1990). For example, oligonucleotides are synthesized by an automated DNA synthesizer (Applied Biosystems Model 380B) using the standard phosphoroamidite chemistry in which oxidation by iodine is carried out, and the apparatuses for carrying out such synthesis are sold by various spellers including Applied Biosystems. Such synthesis of oligonucleotides or analogues thereof may be carried out within the ordinary skill of routine workers. Alternatively, antisense oligonucleotides may be obtained from a number of companies which are specialized in the syntheses of oligonucleotides. Similar techniques for preparing other oligonucleotides such as phosphorothioate derivatives and alkylated derivatives are also well-known. Further, for the syntheses of other modified oligonucleotides such as fluorescence-labeled, biotinylated or cholesterol-modified oligonucleotides, use of similar techniques and use of commercially available modified amidite and CPG product such as biotin, fluorescein, acridine or psoralen-modihed amidite and/or controlled porous glass (CPG) (Glen Research, Sterling VA) is also well-known.

Since antisense method targets the causative gene *per se* of the disease or the disease-related gene *per se*, it is close to causal therapy. Thus, antisense oligonucleotides have a great potential as therapeutic agents for various diseases. For example, U.S. Patent No. 5,135,917 provides an antisense oligonucleotide which inhibits the expression of human interleukin-1 receptor. U.S. Patent No. 5,098,890 relates to an antisense oligonucleotide complementary to c-myb oncogene and to an antisense oligonucleotide therapy of a type of cancerous state. U.S. Patent No. 5,087,617 provides a method for treating cancer patients with an antisense oligonucleotide. U.S. Patent No. 5,166,195 provides an oligonucleotide inhibitor against HIV. U.S. Patent No. 5,004,810 provides an oligomer which can hybridize with mRNA of herpes simplex virus Vmw65 to inhibit replication thereof. U.S. Patent No. 5,194,428 provides an antisense oligonucleotide having anti-viral activity against influenza virus. U.S. Patent No. 4,806,463 provides an antisense oligonucleotide and use thereof to the inhibition of replication of HTLV-III. U.S. Patent No. 5,286,717 relates to a mixed binding oligonucleotide phosphorothioate complementary to an oncogene. U.S. Patent Nos. 5,276,019 and 5,264,423 relate to phosphorothioate oligonucleotide analogues used for the inhibition of replication of foreign nucleic acids. Effectiveness of phosphorothioate oligonucleotides against retinitis by cytomegalovirus in AIDS patients have been shown (Bioworld Today, 1994). Thus, it has been established that oligonucleotides are useful means for therapies, and are useful for the treatment of cells and animals (especially human). Inhibition of expression of casein kinase 2 is also very useful for the therapies of kidney diseases.

Since the cDNA sequences of casein kinase 2 are known (for example, the cDNA sequence of human casein kinase 2 a subunit is described in GenBaak Accession No. J02853, the cDNA sequence of human casein kinase 2 α' subunit is described in GenBank Accession No. M55268, the cDNA sequence of human casein kinase 2 β subunit is described in GenBank Accession No. AY113186, the cDNA sequence of rat casein kinase 2 β subunit is described in GenBank Accession No.NM_031021, the cDNA sequence of rat casein kinase 2 a subunit is described in GenBank Accession No.L15618, the cDNA sequence of mouse casein kinase 2 a subunit is described in GenBank Accession No-AJ001420, and the cDNA sequence of mouse casein kinase 2 Q subunit is described in GenBank Accession No.NM_009975. Among these, the cDNA sequences of human casein kinase 2 α subunit, α' subunit and β subunit, and rat casein kinase 2 a subunit and β subunit are shown in SEQ ID NOs.: 15 to 19, respectively), antisense oligonucleotides against the mRNA transcribed from the casein kinase 2 gene may easily be synthesized.

That is, oligonucleotides having nucleotide sequences complementary to these cDNA sequences may be used as antisense oligonucleotides in principle. However, it is preferred to confirm by BLAST search or the like that the selected nucleotide sequence is specific to the target gene (i.e., the nucleotide sequence or a nucleotide sequence having a high homology thereto does not exist in the genes other than the target gene). For example, as an antisense oligonucleotide against rat casein kinase 2 α subunit (include a' subunit), 5'-gtaatcatcttgattacccca-3' (SEQ ID NO: 1) may be used, and as an antisense oligonucleotide against rat casein kinase 2β subunit, 5'-ggttggccggccgcttgggcc-3' (SEQ ID NO: 2) may be used. In addition, oligonucleotides having nucleotide sequences shown in SEQ ID NOs: 3 to 10 are also exemplified. Further, various oligonucleotides which hybridize with the mRNA of casein kinase 2 are known (e.g., WO 020/62951 and WO/020/62954), and these known oligonucleotides may be used. Nucleotide sequences and parts thereof, as well as analogues thereof, which correspond beyond the species are also useful in the present invention. Not only the coding region of the mRNA, but also other regions such as 5' untranslated region, 3' untranslated region, 5' cap and intron/exon junctions, may be employed as the target region. That is, the oligonucleotide or its analogue prepared in accordance with the present invention may target whole or a part of these regions, similar to the coding region. In a preferred mode, the oligonucleotide or its analogue may specifically hybridize with the transcription initiation site, translation initiation site, or with intron/exon junction sites or nucleotide sequence in the 3' untranslated region. The functions of the mRNA to be interfered include all of the functions for the survival, such as translocation of mRNA to the site of protein translation, actual translation of the protein from mRNA, splicing and maturation of mRNA and independent enzyme activity which is presumably carried out by mRNA. The overall effect of such an interference of the functions of the mRNA is to hinder the expression of the casein kinase 2 protein. Thus, the antisense oligonucleotide or its analogue is the oligonucleotide or its analogue which can at least hybridize with the mRNA coding for the casein kinase 2 protein nucleotide sequence-specifically and which can selectively modulate the expression of casein kinase 2. By administering the antisense oligonucleotide or its analogue to the cells or individual, expression of casein kinase 2 alone may be selectively inhibited nucleotide sequence-specifically, so that the kidney diseases may be cured. The antisense oligonucleotide against casein kinase 2 used in the present invention is an oligonucleotide which has a complementarity and can hybridize with the coding region, 3' untranslated region, 5' untranslated region, 5' cap and/or intron/exon junctions of the mRNA coding for casein kinase 2. By using an oligonucleotide having complementarity to the above-described region, it selectively hybridizes with casein kinase 2. Preferably, the oligonucleotide is an S-oligonucleotide in which at least one binding group in the nucleotide is a sulfur-containing group or phosphorothioate moiety. Pharmaceutical compositions containing an effective amount of at least one antisense oligonucleotide in combination with a pharmaceutically acceptable carrier are also within the scope of the present invention. By administering an antisense oligonucleotide of casein kinase 2, expression of casein kinase 2 may be suppressed, so that kidney diseases may be cured. Especially, it is effective for the diseases characterized by increase of expression of casein kinase 2, such as nephritis. An example is described in detail in the Examples below.

RNAi (RNA interference) method is the method employing the phenomenon that double-stranded RNA administered to cells or individual inhibits the expression of the gene (synthesis of protein) having the same sequence. Since RNAi method selectively suppresses the expression of the target gene by destroying the target gene or mRNA, it attracts attention as a method effective for analysis of function of the gene. Further, since RNAi method targets the causative gene *per se* of the disease or the disease-related gene *per se,* it also attracts attention as a method close to causal therapy. Double-stranded RNAs having 21-23 base units used in RNAi are also called siRNA. siRNAs may easily be designed. For example, to avoid the binding site of transcription factor, the first AA, CA, GA or TA is found from the site downstream of the start codon in the translated region by not less than 50 bases, and the 21 to 23 bases including the subsequent 19 to 21 bases is selected. The selected nucleotide sequence preferably has a GC contents of 30 to 70%, more preferably 50%, and preferably docs not contain GGG or CCC. The it is confirmed that the selected nucleotide sequence is specific to the target gene by BLAST search or the like. Since an siRNA is also a long chain composed of four types of base units, the siRNA against any target gene may be simply and routinely synthesized. By virtue of recent progress, use of siRNA as a novel type of therapeutic method can be devised now. More particularly, as siRNAs for human casein kinase 2β unit, for example, 5'-cuaccgacaagcucuagactt-3' and 5'-gucuagagcuugucgguagtt-3' may be used. Alternatively, for example, 5'-cuaccgacaagcucuagacat--3' and 5'-gucuagagcuugucgguagtg-3' may be used. For rat, 5'--aucuuacuggacucaaugatt--3' and 5'-gauggcuguucgagaucugtt-3', for example, may be used. Alternatively, it is preferred to use an optional similar oligonucleotide or its analogue, which may be prepared by those skilled in the art based on the knowledge of siRNA that is preferred for the inhibition of expression of casein kinase 2, may preferably be used. By administering such an siRNA to cells or individual, expression of casein kinase 2 alone may be selectively inhibited nucleotide scquence-specifically, so that the kidney diseases may be cured. Especially, it is effective for the diseases characterized by increase of expression of casein kinase 2, such as nephritis.

These nucleic acids inhibiting casein kinase 2 may be formulated into a pharmaceutical composition. The pharmaceutical composition may contain a carrier, thickener, vehicle, buffer, preservative, surfactant, liposome or lipid formulation, and may also contain one or more active ingredients such as antibiotics and anesthetic drugs. In addition to such a pharmaceutical carrier, a cationic liquid for easy incorporation of the oligonucleotide, and/or a lipophilic cationic compound which can form liposomes may be contained in the prescription. One of such compositions which attains easy incorporation is lipofectin (BRL, Bethesda MD). Using liposomes for introducing oligonucleotides into cells is described in, for example, U.S. Patent Nos. 4,897,355 and 4,394,448. Further, for general methods for preparing liposomes consisting of biological substances, see U.S. Patent Nos. 4,235,871, 4,231,877, 4,224,179, 4,753,788, 4,673,567, 4,247,411 and 4,814,270. Alternatively, the oligonucleotide may be combined with a lipophilic carrier such as one of a number of sterols including cholesterol, cholic acid and deoxycholic acid. A preferred sterol is cholesterol. Further, the oligonucleotide may be bound to a peptide to be taken by the cells. Examples of the effective peptides include peptide hormones, antigens, antibodies and peptide toxins. By choosing a peptide selectively taken by the cells constituting kidney, specific delivery of the oligonucleotide may be efficiently carried out. The oligonucleotide may be bound via covalent bond through 5' OH group by the formation of an activated aminoalkyl derivative. The selected peptide may then be bound via covalent bond to the oligonucleotide activated by using an amino- and sulfhydryl-reactive hetero bifunctional reagent. The latter binds to cystein residues existing in the peptide. By exposing the cells to the oligonucleotide bound to the peptide, the peptidyl antisense reagent is subjected to endocytosis, and the oligonucleotide binds to the target mRNA to suppress translation. See PCT/US89/02363. The administration dose depends on the severity of the state to be treated and sensitivity, and the treatment is continued for several days to several months until the cure is attained or repression of the discased state is attained. Optimum administration scheme may be calculated based on the measurement of the drug accumulates in the body. Those skilled in the art may easily determine the optimum dose, administration method and repetition frequency. Although the optimum dose may vary depending on the relative effectivity of each oligonucleotide, it may be calculated, in general, based on EC50 determined by animal experiments. For example, once the molecular weight of the compound (determined by the oligonucleotide sequence and chemical structure) and effective dose such as IC50 (experimentally determine) are given, the administration dose in terms of mg/kg is routinely calculated A number of references relate to administration of genes to mammals. Transfer of genes via liposomes and direct transfer of genes to tissues are described in, for example, Brigham et al., (1989) Am. J. Med. Sci., 298:278-281; Nabel et al., (1990) Science, 249:1285-1288; Hajinski et al., (1991) Am. J. Resp. Cell Molec. Biol., 4:206-209; Wang and Huang (1987), Proc. Natl. Acad. Sci., USA., 84:7815-7855. An example of review of human gene therapy is Anderson, Science (1992) 256:808-813.

Although the administration dose of the oligonucleotide is appropriately selected as described above, in most cases, a dose of about 0.005 mg/kg to 5 mg/kg per day in terms of the weight of the oligonucleotide is appropriate. The administration route is preferably parenteral administration such as intravenous administration, intramuscular administration or subcutaneous administration.

The agent for use in therapy and/or prevention of kidney diseases according to the present invention may be used in combination with one or more drugs such as steroids, immunosuppressants, antihypertensive drugs (e.g., calcium blockers, angiotensin converting enzyme inhibitors, ATII receptor blockers and a receptor blocking drugs), antiplatelet drugs (e.g., dipyridamole and dilazep), and anticoagulants. Further, it may be used in combination with gene therapy for introducing HGF or the like. When using these drugs in combination, each drug may be formulated separately or in combination, by mixing with one or more pharmaceutically acceptable carriers, vehicles, binders, diluents and the like, and the resulting pharmaceutical composition may be administered orally or parenterally. In cases where the drugs are separately formulated, the separate formulations may be mixed using a diluent or the like when use and the mixture may be administered, or the separate formulations may be administered to the same subject simultaneously or with a time interval.

As mentioned above, the present inventors discovered that the expression amount of casein kinase 2 gene in the kidney cells of diseased kidney was significantly larger than the expression amount of normal kidney cells. Therefore, the present invention also provides a method for diagnosis of kidney diseases, comprising measuring activity and/or content of casein kinase 2, and/or measuring expression amount of casein kinase 2 gene in a sample separated from body. Here, as the "sample separated from body", cells constituting kidney are preferred.

The diagnostic method of kidney diseases employing the expression of casein kinase 2 as an index may be carried out by using as an index the expression of α subunit, α' subunit and/or β subunit of casein kinase 2, particularly expression of the gene, protein and/or the enzyme activity. For example, in cases where the expression of the gene or protein is used as the index, firstly, RNAs and proteins are extracted from the kidney to be subjected to diagnosis and from a kidney of non-diseased state used as a control. Using a prescribed amount of the extracted RNAs or proteins, expression of casein kinase 2 is detected, respectively. By comparing the expression of casein kinase 2 in the kidney to be diagnosed and the expression in the non-diseased kidney used as a control (healthy individual), the state of the kidney disease in the kidney to be subjected to diagnosis may be diagnosed. If the expression of casein kinase 2 in the kidney subjected to diagnosis is higher than that in the control non-diseased kidney, the kidney subjected to diagnosis is diagnosed as in the diseased condition, especially in the diseased condition involving casein kinase 2. By measuring the expression of casein kinase 2 in the tissue or cells of kidney using such a detection system, the severity of the kidney disease may be measured accurately.

Expression of the casein kinase 2 gene may be measured by using RT-PCR method (Polymerase chain reaction method) ("PCR Protocols" Innis MA, Gelfad DH, Sninsky JJ and White TJ eds., Academic Press, Sandiego (1990)), Northern blot method (Molecular Cloning, Cold Spring Harbor Lab. (1989)), array method, DNA chip method, *in situ* hybridization method, *in situ* RT-PCR (Nucl. Acids Res., 21, 3159-3166(1993)) or the like. Since any of these methods *per se* is well-known, and since the cDNA sequence of casein kinase 2 is also known as mentioned above, those skilled in the art may easily measure the expression of casein kinase 2 gene in the cells, and an example is described in detail in Examples below. Measurement by RT-PCR is preferably employed. That is, from a prescribed amount of the extracted RNAs, cDNAs are synthesized using a reverse transcriptase. By using a prescribed amount of each synthesized cDNA as a template, PCR is performed using primers for casein kinase 2, thereby amplifying the cDNA. A prescribed amount of each PCR product is subjected to agarose gel electrophoresis, and then ethidium bromide staining is performed to detect expression of casein kinase 2 gene. When PCR is performed in relation to the gene of casein kinase 2, it is preferred to carry out PCR in the similar manner using a constantly expressing gene (e.g., a house keeping gene such as G3PDH or β actin) as an internal standard. After subjecting a prescribed amount of the PCR product to agarose gel electrophoresis, ethidium bromide staining is performed to detect the expression of the gene, and the result of the detection of the casein kinase 2 gene is compensated. Any of these steps are conventional. It is preferred to sequence each of the PCR products by a conventional method such as dideoxy method (Proc. Natl. Acad. Sci., USA., 74, 5463(1977)) or Maxam Gilbert method (Methods in Enzymology, 65, 499(1980)), or simply by using a commercially available sequence kit or the like. The primers used for the PCR of casein kinase 2 gene are not restricted as long as they can specifically amplify the gene of each subunit of casein kinase 2, and they may be appropriately designed based on the known nucleotide sequences (*supra*) of the full length cDNAs of these genes. That is, as the sense primer, an oligonucleotide having a nucleotide sequence which is the same as that of the sense chain of cDNA of casein kinase 2 may be employed, and as the antisense primer, an oligonucleotide having a nucleotide sequence which is the same as that of the antisense chain of cDNA of casein kinase 2 may be employed The area sandwiched between the sense primer and the antisense primer is amplified by PCR. The oligonucleotides used as primers may be synthesized by a conventional method using a commercially available chemical synthesizer. The size of the primer is not restricted, and usually about 15 to 30 bases. For example, in case of β subunit of rat casein kinase 2, 5'-ccgcggacataaagatgagt-3' (SEQ ID NO: 11) may be used as the sense primer, and 5'-aaaccagtgccgaagtatgc-3' (SEQ ID NO: 12) may be used as the antisense primer. In case of α subunit of rat casein kinase 2, 5'-agaaagcttcggctaataga-3' (SEQ ID NO: 13) may be used as the sense primer, and 5'-actgaagaaatccctgacat-3' (SEQ ID NO: 14) may be used as the antisense primer. By this, expression of the casein kinase 2 gene may be detected specifically and simply. Alternatively, quantitative PCR such as the so called realtime-detection PCR using a quencher fluorescent dye and a reporter fluorescent dye may also be employed. Since the realtime-detection PCR is also well-known, and kits therefor arc commercially available, it may be easily carried out.

The oligonucleotides according to the present invention may also be used as a probe, and is useful for the detection of the expression of casein kinase 2 gene and for the diagnosis. For example, it may be used as the probe used in the above-described Northern blot method, array method, DNA chip method, *in situ* hybridization method and the like. These methods *per se* are well-known, and those skilled in the art can easily carry out them. A labeled probe obtained by labeling the above-described oligonucleotide with a label such as fluorescent label, radioactive label or biotin label may be used. Whether the test nucleic acid exists in the sample or not may be determined by immobilizing the test nucleic acid or its amplification product on a solid phase, hybridizing the test nucleic acid with the labeled probe, and measuring the label bound to the solid phase after washing. Alternatively, the test nucleic acid may be detected by immobilizing a nucleic acid for measurement on a solid phase, hybridizing the test nucleic acid with the immobilized nucleic acid for measurement, and detecting the test nucleic acid bound to the solid phase with the labeled probe or the like. In such a case, the nucleic acid for measurement immobilized on the solid phase is also called probe. Methods for measuring a test nucleic acid using a nucleic acid probe are also well-known in this field, and may be carried out by contacting the nucleic acid probe with the test nucleic acid in a buffer at Tm or vicinity thereof (preferably within ±4°C) to hybridize them, and after washing, by measuring the hybridized labeled probe or the template nucleic acid bound to the solid phase probe. Such methods include well-known methods such as the above-mentioned Northern blot, *in situ* hybridization and Southern blot method.

For example, an oligonucleotide labeled with a radioactive label may be prepared by labeling the 5' end thereof with ³²P by polynucleotide kinase. Sambrook et al., Molecular Cloning. A Laboratory Manual, Cold Spring Harbor Laboratory Press, 1989, Vol. 2, p10. 59. Then the oligonucleotide labeled with the radioactive label is made to contact with the tissue or cell sample, and then the sample is washed to remove the unbound oligonucleotide. The radioactivity remaining in the sample indicates the bound oligonucleotide (it indicates the expression of casein kinase 2), and the radioactivity may be measured by a scintillation counter or other ordinary means. By performing autoradiography of the tissue using the radioactive labeled oligonucleotide, localization, distribution and/or the amount of the expression of casein kinase 2 gene may be determined. Alternatively, thin section of the tissue may be treated with the radioactive labeled oligonucleotide, and then exposed to photograph emulsion in accordance with the routine autoradiography, after the washing as mentioned above. Upon development, the emulsion gives an image of silver particles in the area in which casein kinase 2 gene is expressed. By quantification of the silver particles, expression of casein kinase 2 may be detected. A similar assay for the detection of casein kinase 2 gene expression by detection of fluorescence may be developed using the oligonucleotide according to the present invention conjugated with fluorescein or other fluorescent label in place of the radioactive label. Each of these modes is known in this field. Those skilled in the art can easily apply these known modes to the detection of expression of casein kinase 2 gene according to the technique of the present invention, thereby novel and useful means for detecting the expression of casein kinase 2 are provided.

In cases where expression of the casein kinase 2 protein is used as an index, casein kinase 2 protein or its partial peptide is detected using a monoclonal antibody and/or polyclonal antibody of which corresponding antigen is a subunit of casein kinase 2 or a partial peptide of casein kinase 2 protein, or antigen-binding fragment thereof, by enzyme-linked immunosorbent assay (ELISA), radio immunoassay (RIA), enzyme immunoassay (EIA), Western blot method, dot blot method, protein chip analysis method, immunostaining analysis method or the like. The antibody or a part thereof used for the detection of casein kinase 2 protein is not restricted as long as it can specifically detect the subunit of casein kinase 2 or a partial peptide of casein kinase 2 protein alone. The polyclonal antibody may be obtained by administering the antigens of casein kinase 2 protein to a rabbit, rat, mouse, goat or the like through parenteral administration, such as intravenous, intramuscular or subcutaneous administration. The monoclonal antibody may be obtained by an ordinary method which is performed by, for example, removing spleen cells of a mouse to which the antigens of casein kinase 2 protein were parenterally administered, fusing the spleen cells with mouse myeloma cells, and purifying the culture supernatant of the fused cells. Casein kinase 2 *per se* may also be prepared easily by a known method. For example, casein kinase 2 may be obtained by extracting casein kinase 2 from an organ such as kidney, liver, spleen, lung, bone marrow, brain or placenta, or from blood cells, of a mammal such as rat or bovine. Alternatively, casein kinase 2 may be obtained by culturing primary cultured cells or established cell line producing casein kinase 2, and separating and purifying casein kinase 2 from the cells. Alternatively, in accordance with a known genetic engineering technique, the gene coding for casein kinase 2 is incorporated into appropriate host cells such as cells of *E coli, Bacillus subtilis,* yeasts, filamentous bacteria, plants and animals, and the desired recombinant casein kinase 2 may be obtained from the culture of the transformants, which may be carried out easily. The above-mentioned various immunoassays and other analysis methods *per se* are well-known. For example, the enzyme-linked immunosorbent assay may be carried out by placing a test sample in a polystyrene microplate on which an anti-casein kinase 2 antibody is immobilized washing the plate after reaction for a prescribed time, adding an anti-casein kinase 2 antibody labeled with an enzyme such as peroxidase, washing the plate again, and adding a substrate such as hydrogen peroxide and a coloring agent such as OPD (*o*-phenylenediamine) to allow coloring. The method for detecting casein kinase 2 in the tissue by the immunostaining analysis method may be carried out by staining isolated tissue with an anti-casein kinase 2 antibody labeled with a fluorescent substance such as FITC (fluorescence isothiocyanate) or with an enzyme label such as peroxidase. According to the present invention, by detecting casein kinase 2 using such a detection system, the severity of the kidney disease may be measured, and this may be carried out easily.

In cases where the expression of enzyme activity of casein kinase 2 is used as the index, the enzyme activity of casein kinase 2 protein or partial polypeptide thereof when the enzyme substrate is made to contact with the casein kinase 2 protein or partial polypeptide thereof is detected, by using protein phosphorylation analysis method, intracellular localization change analysis method or enzyme activity analysis method. In these methods, as the enzyme substrate, any substance which can serve as an enzyme substrate of casein kinase 2 or the peptide, and usually, casein protein or a partial peptide of casein kinase 2 protein is used. Further, as mentioned above, casein kinase 2 may be obtained by various methods. For example, casein kinase 2 may be obtained by extraction and purification from organs of mammals or by separation and purification from cell culture. Alternatively, recombinant casein kinase 2 may be obtained from the culture of the transformants prepared by incorporating the gene coding for casein kinase 2 into appropriate host cells by the known genetic engineering method. The thus obtained casein kinase 2 may be used for confirming the inhibitory activity of the substance which inhibits the enzyme activity of casein kinase 2, even if a part of the amino acid sequence thereof is deleted or substituted, or other amino acid sequence is inserted, as long as it has the enzyme activity. In a method for analyzing the enzyme activity, for example, a sample (10 ng to 0.1 mg protein) is incubated with a peptide Arg-Arg-Arg-Glu-Glu-Glu-Thr-Glu-Glu-Glu or Arg-Arg-Glu-Glu-Glu-Thr-Glu-Glu-Glu (0.2mM) as the enzyme substrate in a buffer (20mM MOPS, pH 72, 25mM β-glycerol phosphate, 5mM EGTA, 1mM sodium o-vanadate, 1 mM dithiothreitol, 15 mM MgCl₂, 01mM [γ-³²P] GTP or [γ-³²P]ATP, 0.002mCi) at 37°C for 10 minutes. After the incubation, 40% trichloroacetic acid is added to stop the reaction, and an aliquot of the reaction solution is taken on phosphocellulose paper (1 to 1.5 cm square, Whatman P81 or the like). The phosphorylation of the peptide which is the enzyme substrate may be measured by washing the phosphocellulose paper with 0.75% phosphoric acid 3 times for 5 minutes per wash, then further washing the paper with acetone for 5 minutes, and measuring the radioactivity of ³²P on the phosphocellulose paper by a scintillation (Econofluor-2, NEN or the like) (R. Roskoski, Methods Enzymol. Vol. 99, pp.3-6, (1983)). High radioactivity of ³²P measured here indicates high expression of the enzyme activity of the casein kinase 2 protein or a partial peptide thereof in the sample. The enzyme activity analysis method similar to this method may be carried out easily because a commercially available enzyme activity-measuring kit (Casein kinase 2 Kinase Assay, Upstate) may be used.

Techniques for the production and purification of the above-described primers and antibodies are well-known in this field. The method for diagnosis of kidney diseases utilizing the expression of casein kinase 2 according to the present invention includes, as mentioned above, the methods detecting expression of casein kinase 2 gene, expression of the protein and expression of the enzyme activity. To detect the gene expression of casein kinase 2, casein kinase 2 gene, primers and/or probe thereto may be used. To detect casein kinase 2, the antibody or a part thereof, to Casein kinase 2 or a partial peptide thereof, or a nucleic acid (aptamer) which specifically recognizes casein kinase 2 may be used. Further, use of an enzyme substrate of casein kinase 2 for the diagnosis of kidney diseases is included. These techniques may be appropriately selected, and by using a reagent kit for the detection of the expression of casein kinase 2, diagnosis of kidney diseases may be carried out simply. Thus, in accordance with the diagnostic method of kidney diseases according to the present invention, diagnostic reagent kits and diagnostic reagents of kidney diseases arc provided.

The method for diagnosis of kidney diseases according to the present invention may be not only applied widely to the diagnoses of human kidney diseases, but also applied to various model animals of kidney diseases. Especially, for glomerulonephritis, findings suggesting the involvement of immunological mechanism have been reported in a number of cases, and preparation of animal experiment models as models of human chronic nephritis has been widely studied. The method of the present invention may preferably be used for the animal models of kidney diseases induced by anti-glomerular basement membrane antibody. That is, by the method of the present invention, whether a kidney disease animal model really suffers from a kidney disease or not may easily be checked. A kidney disease animal model induced by anti-glomerular basement membrane antibody may be prepared by administering an anti-glomerular basement membrane antiserum obtained from rabbit to rat or mouse. The anti-glomerular basement membrane antiserum may be prepared by preparing glomerular basement membrane from renal cortex of rat or mouse, administering the glomerular basement membrane to rabbit together with Freund's complete adjuvant, and obtaining serum ("Method for Screening Pharmacological Effect for Development of New Drugs - Latest Trends and Facts - Vol. 1 ", supervised by Hikaru OZAWA, Shisei Shoin, 1984, p.143). The model is widely used as the most common disease model of primary glomerulonephritis. According to the classification based on morphology and clinical symptoms, it is the disease model closest to crescent-forming glomerulonephritis and acute progressive glomerulonephritis, among the primary glomerulonephritis. The diseased state may be grasped by measuring urinary protein excretion which is a kidney function marker, blood creatinine level or endogenous creatinine clearance. Since creatinine is not absorbed by kidney at all, if the filtering function of the glomeruli is deteriorated by a kidney disease, the blood creatinine level is increased. Therefore, blood creatinine level and creatinine clearance are good markers of kidney function. In the second week from the induction of nephritis, the blood creatinine, level is increased twice of the normal value, and the animal present the diseased state of nephritis and renal failure accompanying the general decrease in the filtration ability of glomeruli. Such an animal model of kidney diseases, produced by induction by anti-glomerular basement membrane antibody, is widely used in the world as the model of human chronic nephritis. The animal model may be used by the method of Yoshio SUZUKI et al. (Journal of Japan Society of Nephrology, Vol. 23, pp.323-331, 1981) and (Journal of Japan Society of Nephrology, Vol. 77, pp.407-417, 1981). The utility of the drug according to the present invention may also be confirmed. Other useful animal models of kidney diseases include 1) those prepared by Thy-1 monoclonal antibody or anti-thymus antibody, 2) hereditary nephrosis rats and mice, 3) spontaneous diabetes models of mice and rats, 4) diabetes models induced by administering streptozotocin or alloxan to mice and rats, and 5) models from which 5/6 of the kidney was resected.

The present invention will be described more concretely by way of examples below.

### Example 1

The relationship between the expression of casein kinase 2 in the kidney tissue, that is, in the cells constituting the kidney and the state of the kidney disease was analyzed. More particularly, using rats, the state of the kidney disease was diagnosed using the urinary protein excretion which is a widely used kidney function marker, and the expression of casein kinase 2 a subunit in the kidney tissue was detected by RT-PCR method, so as to analyze the relationship between the state of the kidney disease and the expression of casein kinase 2.

To rats (Wistar-Kyoto strain, male, body weight 190 to 210 g, Charles River Japan, Inc.), rabbit anti-glomerular basement membrane antibody was intravenously administered (0.3 ml/kg) to induce nephritis. To the rats of normal group, normal rabbit serum (0.3 ml/kg) was intravenously administered. On 7 to 14 days after the induction of nephritis, all rats were placed in metabolic cages, and urine excreted during 24 hours was collected and the amount of the urine was measured. The urine was then centrifuged at 3000 rpm for 15 minutes, and the urine protein in the supernatant was measured using TP Test Wako (Wako Pure Chemical). Further, after the collection of urine, the kidneys were isolated, and RNAs were extracted therefrom by a conventional method, followed by preparation of cDNAs from 1 µg of RNAs using a commercially available reverse transcription reaction kit (Invitrogen). Using 1 µl each of the synthesized cDNAs as templates, PCR was performed using the primers for casein kinase 2 α subunit and using a commercially available PCR kit (Takara) to amplify the cDNA. Primers were prepared based on the nucleotide sequence of the full length cDNA of rat casein kinase 2 α subunit. That is, as the sense primer, 5'-agaaagcucggctaataga-3' was used, and as the antisense primer, 5'-actgaagaaatccctgacat-3' was used. The PCR was carried out by repeating 30 times the thermal cycle of 94°C for 30 seconds, 58°C for 30 seconds and 72°C for 40 seconds, and the reaction was stopped finally at 4°C. Each of 10 µl aliquots of the amplified PCR products was electrophoresed on agarose gel, and the gel was subjected to ethidium bromide staining, followed by measuring the expression of the casein kinase 2 α subunit gene. This measurement was carried out by taking photographs of the detected casein kinase 2 so as to make image files of the expression of the casein kinase 2 gene, and degitalizating the image file using a software NIH Image on Macintosh. When the PCR was carried out, a constantly expressing gene (a house keeping gene, G3PDH) was detected as an internal standard, and the results of detection of the expression of casein kinase 2 a subunit gene was compensated.

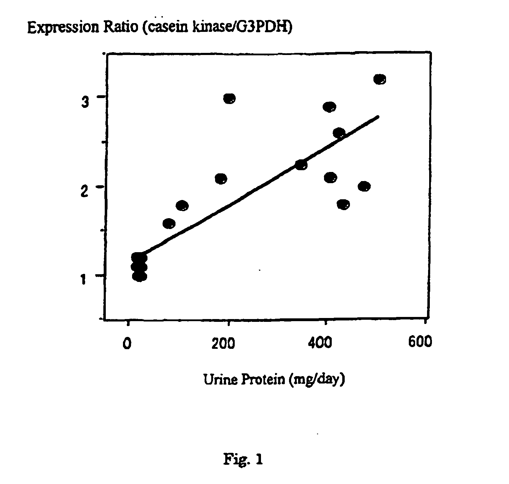

As a result, as shown in Fig. 1, the expression amount of casein kinase 2 α subunit gene in the kidney tissue, i.e., in the cells constituting the kidney in the diseased state, is prominently larger than that in the normal kidney. Further, it can be easily seen that the larger the expression amount of the casein kinase 2 gene in the cells constituting the kidney, the worse the state of the kidney disease, and the closer the expression amount to the normal level, the closer the state of the kidney disease to the normal state.

### Example 2

Using the expression of casein kinase 2α subunit gene as an index, kidney disease was diagnosed. That is, using rats, expression of casein kinase 2α subunit gene in the kidney tissue, i.e., in the cells constituting the kidney, was detected by RT-PCR method, and the kidney disease was diagnosed using it as an index.

First, to rats (Wistar-Kyoto strain, male, body weight 190 to 210g, Charles River Japan, Inc.), rabbit anti-glomerular basement membrane antibody was intravenously administered (0.3 ml/kg) to induce nephritis. To the rats of normal group, normal rabbit serum (0.3 ml/kg) was intravenously administered. After the induction of nephritis, all rats were placed in metabolic cages, and urine excreted during 24 hours was collected, and then the kidneys were isolated. RNAs were extracted from each kidney to be subjected to diagnosis and from each kidney of non-diseased state as a control by a conventional method, and cDNAs were synthesized from 1 µg of the respective RNAs using a commercially available reverse transcription reaction kit (Invitrogen). Using 1 µl each of the synthesized cDNAs as templates, PCR was performed using the primers for casein kinase 2 α subunit and using a commercially available PCR kit (Takara) to amplify the cDNA. Primers were prepared based on the nucleotide sequence of the full length cDNA of rat casein kinase 2 a subunit. That is, as the sense primer, 5'-agaaagcttcggctaataga-3' was prepared and used, and as the antisense primer, 5'-actgaagaaatccctgacat-3' was prepared and used. The PCR was carried out by repeating 30 times the thermal cycle of 94°C for 30 seconds, 58°C for 30 seconds and 72°C for 40 seconds, and the reaction was stopped finally at 4°C. Each of 10 µl aliquots of the amplified PCR products was electrophoresed on agarose gel, and the gel was subjected to ethidium bromide straining, followed by measuring the expression of the casein kinase 2 gene. This measurement was carried out by taking photographs of the detected casein kinase 2 so as to make image files of the expression of the casein kinase 2 gene, and degitalizating the image file using a software NIH Image on Macintosh. When the PCR was carried out, a constantly expressing gene (a house keeping gene, G3PDH) was detected as an internal standard, and the results of detection of the expression of casein kinase 2α subunit gene was compensated.

Expression of the casein kinase 2α subunit gene in the kidney subjected to diagnosis, and the expression of the casein kinase 2α subunit gene in the non-diseased kidney used as a control were compared. As a result, expression of the casein kinase 2 a subunit gene in the kidney subjected to diagnosis was higher than that in the non-diseased kidney used as a negative control (Table 1). Therefore, the kidney subjected to diagnosis was diagnosed to be in the diseased condition, especially in the diseased condition involving casein kinase 2. To verify these diagnosis results, the urinary protein excretion which is a widely used kidney function marker of each rat individual diagnosed as in the diseased condition was measured. The urinary protein excretion of each non-diseased individual used as a control was also measured. As a result, the urinary protein amounts of the individuals diagnosed as in the kidney-diseased state were clearly larger than those of the individuals of non-diseased state (Table 2). Thus, it was confirmed that the individuals diagnosed as in the kidney-diseased state were actually in the kidney-diseased state, and it was proved that the diagnostic method according to the present invention can accurately judge the diseased state of the kidney disease.

**Table 1**

| | |
|---|---|
| Results of Detection of Expression of Casein Kinase 2 a Subunit Gene When Kidney Disease was Diagnosed Using Expression of Casein Kinase 2 a Subunit Gene as Index are Shown in Terms of Values. | |

| Kidney | Expression Ratio (casein kinase 2/G3PDH) |
|---|---|
| Kidney Subjected to Diagnosis | 3.4 |
| Non-diseased Kidney | 1.1 |

**Table 2**

| | |
|---|---|
| Results of Measurement of Urine Protein of Individuals Diagnosed as in Kidney-diseased State Using Expression of Casein Kinase 2α Subunit Gene as Index | |

| Individual | Urine Protein (mg/day) |
|---|---|
| Individual Diagnosed as in Kidney-diseased State | 142.9 |
| Individual Not in Kidney-diseased State | 28.7 |

### Example 3

Using the expression of casein kinase 2β subunit gene as an index, kidney disease was diagnosed. That is, using rats (Wistar-Kyoto strain, male, body weight 190 to 210 g, Charles River Japan, Inc.), expression of casein kinase 2β subunit gene in the kidney tissue, that is, in the cells constituting the kidney, was detected by RT-PCR method, and the kidney disease was diagnosed using it as an index.

First, to rats, rabbit anti-glomerular basement membrane antibody was intravenously administered (0.3 ml/kg) to induce nephritis. To the rats of normal group, normal rabbit serum (0.3 ml/kg) was, intravenously administered. After the induction of nephritis, all rats were placed in metabolic cages, and urine excreted during 24 hours was collected, and then the kidneys were isolated. RNAs were extracted from each kidney to be subjected to diagnosis and from each kidney of non-diseased state as a control by a conventional method, and cDNAs were synthesized from 1 µg of the respective RNAs using a commercially available reverse transcription reaction kit (Invitrogen). Using 1 µl each of the synthesized cDNAs as templates, PCR was performed using the primers for casein kinase 2β subunit and using a commercially available PCR kit (Takara) to amplify the cDNA. Primers were prepared based on the nucleotide sequence of the full length cDNA of rat casein kinase 2β subunit. That is, as the sense primer, 5'-ccgcggacataaagatgagt-3' was prepared and used, and as the antisence primer, 5'-aaaccagtgccgaagtatgc-3' was prepared and used. The PCR was carried out by repeating 30 times the thermal cycle of 94°C for 30 seconds, 58°C for 30 seconds and 72°C for 40 seconds, and the reaction was stopped finally at 4°C. Each of 10 µl aliquots of the amplified PCR products was electrophoresed on agarose gel, and the gel was subjected to ethidium bromide staining, followed by measuring the expression of the casein kinase 2 gene. This measurement was carried out by taking photographs of the detected casein kinase 2 so as to make image files of the expression of the casein kinase 2 gene, and degitalizating the image file using a software NIH Image on Macintosh. When the PCR was carried out, a constantly expressing gene (a house keeping gene, G3PDH) was detected as an internal standard, and the results of detection of the expression of casein kinase 2 β subunit gene was compensated.

Expression of the casein kinase 2 β subunit gene in the kidney subjected to diagnosis, and the expression of the casein kinase 2 β subunit gene in the non-diseased kidney used as a control were compared. As a result, expression of the casein kinase 2 β subunit gene in the kidney subjected to diagnosis was higher than that in the non-diseascd kidney used as a negative control (Table 3). Therefore, the kidney subjected to diagnosis was diagnosed to be in the diseased condition, especially in the diseased condition involving casein kinase 2. To verify these diagnosis results, the urinary protein excretion which is a widely used kidney function marker of each rat individual diagnosed as in the diseased condition was measured. The urinary protein excretion of each non-diseased individual used as a control was also measured. As a result, the urinary protein amounts of the individuals diagnosed as in the kidney-diseased state were clearly larger than those of the individuals of non-diseased state (Table 4). Thus, it was confirmed that the individuals diagnosed as in the kidney-diseased state were actually in the kidney-diseased state, and it was proved that the diagnostic method according to the present invention can accurately judge the diseased state of the kidney disease.

**Table 3**

| | |
|---|---|
| Results of Detection of Expression of Casein Kinase 2 β Subunit Gene When Kidney Disease was Diagnosed Using Expression of Casein Kinase 2 β Subunit Gene as Index are Shown in Terms of Values. | |

| Kidney | Expression Ratio (casein kinase 2/G3PDH) |
|---|---|
| Kidney Subjected to Diagnosis | 4.5 |
| Non-diseased Kidney | 1.4 |

**Table 4**

| | |
|---|---|
| Results of Measurement of Urine Protein of Individuals Diagnosed as in Kidney-diseased State Using Expression of Casein Kinase 2 β Subunit Gene as Index | |

| Individual | Urine Protein (mg/day) |
|---|---|
| Individual Diagnosed as in Kidney-diseased State | 146.2 |
| Individual Not in Kidney-diseased State | 37.4 |

### Example 4

Using the expression of casein kinase 2 as an index, kidney disease was diagnosed. That is, using diabetic rats (Zucker strain) which spontaneously suffered from diabetic nephropathy, expression of casein kinase 2 β subunit gene in the kidney was detected by RT-PCR method, and the kidney disease was diagnosed using it as an index. Blood was sampled from the rats which spontaneously suffered from diabetic nephropathy (Zucker fa/fa strain, 6 months old, Charles River Japan, Inc.) and from the control rats which did not spontaneously suffered from diabetic nephropathy (Zucker Lean strain, 6 months old, Charles River Japan, Inc.), and then kidneys were isolated from the rats. From each kidney to be subjected to diagnosis and from each control kidney which was not diseased, RNAs were extracted by a conventional method, and cDNAs were synthesized from 1 µg of the respective RNAs using a commercially available reverse transcription reaction kit (Invitrogen). Using 1 µl each of the synthesized cDNAs as templates, PCR was performed using the primers for casein kinase 2 α subunit and using a commercially available PCR kit (Takara) to amplify the cDNA. Primers were prepared based on the nucleotide sequence of the full length cDNA of rat casein kinase 2 β subunit. That is, as the sense primer, 5'-ccgcggacataaagatgagt-3' was prepared and used, and as the antisense primer, 5'-aaaccagtgccgaagtatge-3' was prepared and used. The PCR was carried out by repeating 30 times the thermal cycle of 94°C for 30 seconds, 58°C for 30 seconds and 72°C for 40 seconds, and the reaction was stopped finally at 4°C. Each of 10 µl aliquots of the amplified PCR products was electrophoresed on agarose gel, and the gel was subjected to cthidium bromide staining, followed by measuring the expression of the casein kinase 2 gene. This measurement was carried out by taking photographs of the detected casein kinase 2 so as to make image files of the expression of the casein kinase 2 gene, and degitalizating the image file using a software NIH Image on Macintosh. When the PCR was carried out, a constantly expressing gene (a house keeping gene, G3PDH) was detected as an internal standard, and the results of detection of the expression of casein kinase 2 β subunit gene was compensated.

Expression of the casein kinase 2 β subunit gene in the kidney subjected to diagnosis, and the expression of the casein kinase 2 β subunit gene in the non-diseased kidney used as a control were compared. As a result, expression of the casein kinase 2 β subunit gene in the kidney subjected to diagnosis was higher than that in the non-diseased kidney used as a negative control (Table 5). Therefore, the kidney subjected to diagnosis was diagnosed as in the diseased condition, especially in the diseased condition involving casein kinase 2. To verify these diagnosis results, the blood creatinine level and the blood urea nitrogen, which are widely used kidney function markers, of each rat individual diagnosed as in the diseased condition were measured. Those of each non-diseased individual used as a control were also measured. As a result, the blood creatinine levels of the individuals diagnosed as in the kidney-diseased state were clearly larger than those of the individuals of non-diseased state (Table 6). Further, the blood urea nitrogen of the individuals diagnosed as in the kidney-diseased state was clearly larger than those of the individuals of non-diseased state (Table 6). Thus, it was confirmed that the individuals diagnosed as in the kidney-diseased state were actually in the kidney-diseased state, and it was proved that the diagnostic method according to the present invention can accurately judge the diseased state of the kidney disease.

**Table 5**

| | |
|---|---|
| Results of Detection of Expression of Casein Kinase 2 Gene When Kidney Disease was Diagnosed Using Expression of Casein Kinase 2 Gene as Index are Shown in Terms of Values. | |

| Kidney | Expression Ratio (casein kinase 2/C3PDH) |
|---|---|
| Kidney Subjected to Diagnosis | 3.6 |
| Non-diseased Kidney | 1.0 |

**Table 6**

| | | |
|---|---|---|
| Results of Measurement of Blood Creatinine Level and Blood Urea Nitrogen of Individuals Diagnosed as in Kidney-diseased State Using Expression of Casein Kinase 2 Gene as Index | | |

| Individual | Blood Creatinine Level (mg/100 ml) | Blood Urea Nitrogen (mg/100ml) |
|---|---|---|
| Individual Diagnosed as in Kidney-diseased State | 0.89 | 69.0 |
| Individual Not in Kidney-diseased State | 0.38 | 18.3 |

### Example 5

Using the content of casein kinase 2 as an index, kidney disease was diagnosed. That is, using rats, the amounts of the casein kinase 2 α subunit protein and β subunit protein in the kidney were detected using Western blot method, and kidney disease was diagnosed using these amounts.

First, to rats (Wistar-Kyoto strain, male, body weight 190 to 210 g, Charles River Japan, Inc.), rabbit anti-glomerular basement membrane antibody was intravenously administered (0.3 ml/kg) to induce nephritis. To the rats of normal group, normal rabbit serum (0.3 ml/kg) was intravenonsly administered. After the induction of nephritis, all rats were placed in metabolic cages, and urine excreted during 24 hours was collected, and then the kidneys were isolated. From each kidney to be subjected to diagnosis and from each control kidney which was not diseased, proteins were extracted by a conventional method, and the proteins (1 mg/ml, 20 µl) were electrophoresed (PAGERUN, ATTO, 40mA, 84min) on 12.5% polyacrylamide gel (PAGEL, ATTO). The electrophoresed proteins were blotted (100V, 90 min) on a PVDF membrane (Millipore). The membrane was blocked (Blocking Ace, Dainippon Pharmaceutical, 4°C, 1 hour), and washed three times (10 min) with PBS containing Tween 20. Then the membrane was incubated (at room temperature for 1 hour) with an anti-casein kinase 2 α subunit (originated from goat, Santa cruz, 100-fold diluted) or an anti-casein kinase 2 β subunit (originated from goat, Santa cruz, 100-fold diluted) as the primary antibody. After the incubation and after washing (10 min) the membrane 3 times with PBS containing Tween 20, the membrane was incubated (at room temperature for 1 hour) with an anti-goat IgG antibody labeled with HRP (Santa cruz, 1000-fold diluted) used as the secondary antibody. After the incubation, the amounts of the casein kinase 2 α subunit protein and the casein kinase 2 β subunit protein were measured, respectively, using ECL reagent (Amersham). This measurement was carried out by taking photographs of the detected casein kinase 2 so as to make image files of the amount of the casein kinase 2 protein, and degitalizating the image file using a software NIH Image on Macintosh.

The amount of casein kinase 2 a subunit protein in the kidney subjected to diagnosis, and the amount of casein kinase 2 a subunit protein in the non-diseased kidney used as a control were compared. As a result, the amount of casein kinase 2 α subunit protein in the kidney subjected to diagnosis was higher than that in the non-diseased kidney used as a negative control (Table 7). The amount of casein kinase 2 β subunit protein in the kidney subjected to diagnosis, and the amount of casein kinase 2 β subunit protein in the non-diseased kidney used as a control were compared. As a results, the amount of casein kinase 2 β subunit protein in the kidney subjected to diagnosis was higher than that in the non-diseased kidney used as a negative control (Table 8). Therefore, the kidney subjected to diagnosis was diagnosed to be in the diseased condition, especially in the diseased condition involving casein kinase 2. To verify these diagnosis results, the urinary protein excretion which is a widely used kidney function marker of each rat individual diagnosed as in the diseased condition was measured. The urinary protein excretion of each non-diseased individual used as a control was also measured. As a result, the urinary protein amounts of the individuals diagnosed as in the kidney-diseased state were clearly larger than those of the individuals of non-diseased state (Table 9). Thus, it was confirmed that the individuals diagnosed as in the kidney-diseased state were actually in the kidney-diseased state, and it was proved that the diagnostic method according to the present invention can accurately judge the diseased state of the kidney disease. Further, it can be easily seen that the larger the expression amount, i.e., the content of the casein kinase 2 in the cells constituting the kidney, the worse the state of the kidney disease, and the closer the expression amount, i.e., the content, to the normal level, the closer the state of the kidney disease to the normal state.

**Table 7**

| | |
|---|---|
| Results of Detection of Amount of Casein Kinase 2 α Subunit When Kidney Disease was Diagnosed Using Content of Casein Kinase a Subunit Protein as Index are Shown in Terms of Values. | |

| Kidney | Protein Content Ratio of Casein Kinase 2α Subunit |
|---|---|
| Kidney Subjected to Diagnosis | 2.2 |
| Non-diseased Kidney | 1.0 |

**Table 8**

| | |
|---|---|
| Results of Detection of Amount of Casein Kinase β Subunit When Kidney Disease was Diagnosed Using Content of Casein Kinase β Subunit Protein as Index are Shown in Terms of Values. | |

| Kidney | Protein Content Ratio of Casein Kinase 2β Subunit |
|---|---|
| Kidney Subjected to Diagnosis | 2.4 |
| Non-diseased Kidney | 1.0 |

**Table 9**

| | |
|---|---|
| Results of Measurement of Urine Protein of Individuals Diagnosed as in Kidney-diseased State Using Contents of Casein Kinase 2 a Subunit and β Subunit as Indices | |

| Individual | Urine Protein (mg/day) |
|---|---|
| Individual Diagnosed as in Kidney-diseased State | 151.7 |
| Individual Not in Kidney-diseased State | 38.3 |

### Example 6

The relationship between casein kinase 2 protein content and enzyme activity was analyzed. That is, each of the casein kinase 2 (Upstate) having varying protein contents (0, 10 and 20 ng protein, respectively) was incubated in 0.05 ml of a buffer (20mM MOPS, pH7.2, 25mM β-glycerose phosphate, 5mM EGTA,1 mM sodium o-vanadate. 1mM dithiothreitol, 15mM MgCl₂, 0.1 mM [γ-³²P]ATP, 0.002 mCi, Amersham) containing Arg-Arg-Arg-Glu-Glu-Glu-Thr-Glu-Glu-Glu (10nmol, 0.2mM, Upstate) which is an enzyme substrate, at 37°C for 10 minutes. After the incubation, 0.025 ml of 40% trichloroacetic acid was added to stop the reaction, and 0.025 ml aliquot of the reaction solution was taken on phosphocellulose paper (1 cm x 1 cm). The phosphocellulose paper was washed three times with 25 ml of 0.75% phosphoric acid for 5 minutes per wash, and then washed with 25 ml of acetone for 5 minutes, followed by measurement of the radioactivity of ³²P on the phosphocellulose paper by scintillation.

As a result, as shown in Table 10, the larger the casein kinase 2 protein content, the higher the enzyme activity, i.e., the function of casein kinase 2, and it can be easily seen that the expression amount, i.e., content of casein kinase 2 protein, is closely related to the enzyme activity, i.e., function of casein kinase 2. These indicate that the larger the expression amount of casein kinase 2 gene of casein kinase 2 protein, i.e., the content of the casein kinase 2 protein, the worse the state of the kidney disease, and the closer these values to the normal levels by suppression of these, the closer the state of the kidney disease to the normal state.

**Table 10**

| | |
|---|---|
| Relationship between Casein Kinase 2 Protein Content and Enzyme Activity | |

| Amount of Protein (ng) | Enzyme Activity (cpm) |
|---|---|
| 0 | 4,511 |
| 10 | 46,751 |
| 20 | 92,203 |

### Example 7

This example was carried out for studying the effect of administration of an antisense oligonucleotide against casein kinase 2 a subunit, so as to confirm the utility of the present invention. To rats (Wistar-Kyoto strain, male, body weight 190 to 210 g), rabbit anti-glomerular basement membrane antibody was intravenously administered (0.3 ml/kg) to induce nephritis. To the kidney tissues of the nephritis rats, an antisense Oligonucleotide 5'-GTAATCATCTTGATTACCCCA-3' which selectively inhibits expression of casein kinase 2 a subunit, or a sense oligonucleotide 5'-TGGGGTATCAATCAACATGATTAC-3' which does not inhibit the expression of casein kinase 2 α subunit was administered at a dose of 12 µg/day which is said to be appropriate for the inhibition of casein kinase 2 using Alzet pumps (0.25 µl/hour, 1002, Alzet) connected through polyethylene tubes(PE-10), continuously from one day before the induction of nephritis to the 7th day. The nucleic acid molecules of the oligonucleotides were S-oligonucleotides containing phosphorothioate moieties, and a cationic liquid (Polyplus transfection) was used for making the uptake of the oligonucleotide easier. More particularly, the groups used in the experiment were normal group (normal rats to which the antisense oligonucleotide against casein kinase 2 α subunit and the antisense oligonucleotide were not administered, n=8), nephritis control group (nephritis rats to which the antisense oligonucleotide against casein kinase 2 α subunit and the antisense oligonucleotide were not administered, n=8), nephritis + antisense oligonucleotide against casein kinase 2 a subunit-administered group (group of the present invention, nephritis rats to which the antisense oligonucleotide against casein kinase 2 a subunit was administered, n=4), and nephritis + sense oligonucleotide of casein kinase 2 α subunit-administered group (negative control group, nephritis rats to which the sense oligonucleotide of casein kinase 2 α subunit was administered), totally four groups. On the 7th day from the induction, all rats were placed in metabolic cages, and urine excreted during 24 hours was collected and the amount of the urine was measured. The urine was then centrifuged at 3000 rpm for 15 minutes, and the urine protein in the supernatant was measured. Further, after the collection of urine, the kidneys were isolated, proteins were extracted from each kidney tissue by a conventional method, and the proteins (1 mg/ml, 20 µl) were electrophoresed (PAGERUN, ATTO, 40mA, 84min) on 12.5% polyacrylamide gel (PAGEL, ATTO). The electrophoresed proteins were blotted (100V, 90 min) on a PVDF membrane (Millipore). The membrane was blocked (Blocking Acc, Dainippon Pharmaceutical 4°C, 1 hour), and washed three times (10 min) with PBS containing Tween 20. Then the membrane was incubated (at room temperature for 1 hour) with an anti-casein kinase 2 a subunit (originated from goat, Santa cruz, 100-fold diluted) as the primary antibody. After the incubation and after washing (10 min) the membrane 3 times with PBS containing Tween 20, the membrane was incubated (at room temperature for 1 hour) with an anti-goat IgG antibody labeled with HRP (Santa cruz, 1000-fold diluted used as the secondary antibody). After the incubation, the amounts of the casein kinase 2 α subunit protein were measured, respectively, using ECL reagent (Amersham). This measurement was carried out by taking photographs of the detected casein kinase 2 so as to make image files of the amount of the casein kinase 2 protein, and degitalizating the image file using a software NIH Image on Macintosh.

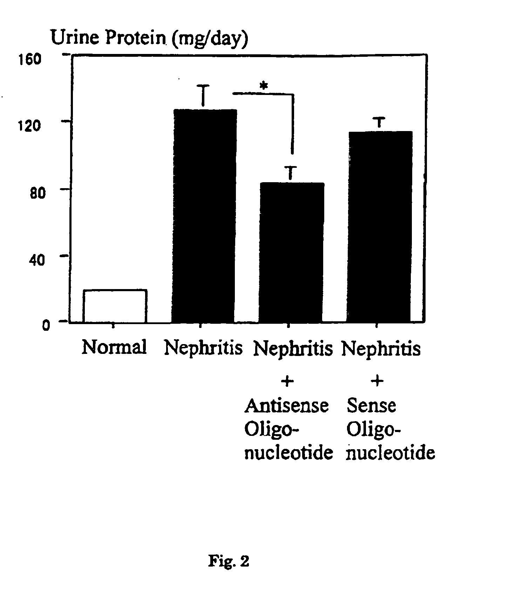

The results are shown in Table 11 and Fig. 2. The antisense oligonucleotide against casein kinase 2 a subunit according to the present invention prominently decreased the expression amount, i.e. the content, of the casein kinase 2 a subunit protein in the kidney tissue, i.e., in the cells constituting the kidney (Table 11), and significantly decreased the urine protein which is an important marker of the diseased state of kidney (Fig. 2). On the other hand, the sense oligonucleotide of casein kinase 2 α subunit which was the negative control did not decrease the expression amount, i.e. the content, of the casein kinase 2 α subunit protein in the kidney tissue, i.e., in the cells constituting the kidney (Table 11), and did not significantly decrease the urine protein which is an important marker of the diseased state of kidney (Fig. 2). Thus, these facts give a generally recognizable theoretical ground that means and substances which decrease or inhibit the expression of the casein kinase 2 in the kidney tissue, i.e., in the cells constituting the kidney, have actions or effects to make the state of the kidney disease close to the normal state, so that they have actions or effects for curing and preventing kidney diseases. Since no difference in body weight was observed between the antisense oligonucleotide against casein kinase 2-administered group and the nephritis control group, and between the antisense oligonucleotide against casein kinase 2-administered group and the negative control group, and since no damage was observed in organs, it was proved that the antisense oligonucleotide is safely used as a therapeutic agent for kidney diseases.

**Table 11**

| | |
|---|---|
| Effects of Administration of Antisense Oligonucleotide or Sense Oligonucleotide of Casein Kinase 2 a Subunit on Amount of Casein Kinase 2 α Subunit Protein in Kidney Tissues in Nephritis Rat Models | |

| Kidney | Protein Amount Ratio of Casein Kinase 2 α Subunit |
|---|---|
| Antisense Oligonucleotide-administered Kidney | 0.45 |
| Sense Oligonucleotide-administered Kidney | 1.07 |
| No Oligonucleotide-administered Kidney | 1 |

### Industrial Availability

The agent for use in therapy and/or prevention of kidney diseases according to the present invention may be used for the therapy and/or prevention of kidney diseases. By the diagnostic (detection) method according to the present invention, kidney diseases may be diagnosed (detected) accurately and simply.

## Claims

1. An agent for use in therapy and/or prevention of kidney diseases, comprising as an effective ingredient a nucleic acid molecule that inhibits expression of casein kinase 2.

2. The agent for use in therapy and/or prevention of kidney diseases according to claim 1, wherein said casein kinase 2 is originated from cells constituting kidney.

3. The agent for use in therapy and/or prevention of kidney diseases according to claim 1 or 2, wherein said nucleic acid molecule is a nucleic acid molecule which inhibits expression of α subunit and/or α' subunit of casein kinase 2.

4. The agent for use in therapy and/or prevention of kidney diseases according to any one of claims 1 to 3, wherein said nucleic acid molecule which inhibits expression of casein kinase 2 is an antisense oligonucleotide that targets mRNA coding for casein kinase 2, and that can inhibit expression of casein kinase 2.

5. The agent for use in therapy and/or prevention of kidney diseases according to any one of claims 1 to 4, wherein said antisense oligonucleotide has 12 to 50 bases having a sequence selected from the group consisting of coding regions, 3'-untranslated regions, 5'-untranslated region, 5' cap and intron/exon junctions of mRNA coding for casein kinase 2.

6. The agent for use in therapy and/or prevention of kidney diseases according to any one of claims 1 to 5, wherein said nucleic acid molecule of said antisense oligonucleotide against casein kinase 2 is an S-oligonucleotide.

7. The agent for use in therapy and/or prevention of kidney diseases according to any of claims 1 to 6, wherein said nucleic acid molecule forming said antisense oligonucleotide against casein kinase 2 has a sequence shown in any one of SEQ ID NOs: 1-10.

8. The agent for use in therapy and/or prevention of kidney diseases according to any one of claims 1 to 7, wherein said antisense oligonucleotide against casein kinase 2 has a sequence shown in SEQ ID NO: 1.

9. The agent for use in therapy and/or prevention of kidney diseases according to any one of claims 1 to 8, wherein said kidney disease is glomerular nephritis, interstitial nephritis, nephrosclerosis, diabetic nephropathy or chronic or acute renal failure.

10. The agent for use in therapy and/or prevention of kidney diseases according to any one of claims 1 to 9, wherein said kidney disease is a nephritis other than one resulted from diabetes, or said kidney disease is **characterized by** increase in expression of casein kinase 2 or increase in enzyme activity of casein kinase 2.

11. A method for diagnosis of kidney diseases, comprising measuring activity and/or content of casein kinase 2, and/or measuring expression amount of casein kinase 2 gene in a sample separated from body.

12. The method according to claim 11, wherein said sample is cells constituting kidney.

13. The method according to claim 11 or 12, wherein said casein kinase 2 is α subunit and/or α' subunit.

14. The method according to any one of claims 11 to 13, wherein said kidney disease is glomerular nephritis, interstitial nephritis, nephrosclerosis, diabetic nephropathy or chronic or acute renal failure.

15. The method according to any one of claims 11 to 13, wherein said kidney disease is a nephritis other than one resulted from diabetes, or said kidney disease is **characterized by** increase in expression of casein kinase 2 or increase in enzyme activity of casein kinase 2.

16. The method according to any one of claims 11 to 15, comprising measuring expression amount of casein kinase 2 gene.

17. An oligonucleotide having a nucleotide sequence complementary to a region in sense chain or antisense chain of casein kinase 2 gene, which is used for the method according to any one of claims 11 to 16.

18. The oligonucleotide according to claim 17, which is a primer for gene amplification or a probe for detecting gene.

## Patentansprüche

1. Mittel zur Verwendung in der Therapie und/oder der Prävention von Nierenkrankheiten, welches als wirksamen Bestandteil ein Nukleinsäuremolekül umfasst, das die Expression von Casein Kinase 2 inhibiert.

2. Mittel zur Verwendung in der Therapie und/oder der Prävention von Nierenkrankheiten gemäß Anspruch 1, wobei die Casein Kinase 2 von Zellen stammt, aus denen die Niere aufgebaut ist.

3. Mittel zur Verwendung in der Therapie und/oder der Prävention von Nierenkrankheiten gemäß Anspruch 1 oder 2, worin das Nukleinsäuremolekül ein Nukleinsäuremolekül ist, das die Expression der α-Untereinheit und/oder der α'-Untereinheit von Casein Kinase 2 inhibiert.

4. Mittel zur Verwendung in der Therapie und/oder der Prävention von Nierenkrankheiten gemäß irgendeinem der Ansprüche 1 bis 3, worin das Nukleinsäuremolekül, das die Expression von Casein Kinase 2 inhibiert, ein Antisense-Oligonukleotid ist, das auf mRNA gerichtet ist, die für Casein Kinase 2 kodiert und das die Expression von Casein Kinase 2 inhibieren kann.

5. Mittel zur Verwendung in der Therapie und/oder der Prävention von Nierenkrankheiten gemäß irgendeinem der Ansprüche 1 bis 4, worin das Antisense-Oligonukleotid 12 bis 50 Basen hat, die eine Sequenz haben, die ausgewählt ist aus der Gruppe bestehend aus kodierenden Bereichen, 3'-untranslatierten Bereichen, 5'-untranslatiertem Bereich, 5'-Cap und Intron-/Exon-Verbindungen von mRNA, die für Casein Kinase 2 kodiert.

6. Mittel zur Verwendung in der Therapie und/oder der Prävention von Nierenkrankheiten gemäß irgendeinem der Ansprüche 1 bis 5, worin das Nukleinsäuremoleküldes Antisense-Oligonukleotids gegen Casein Kinase 2 ein S-Oligonukleotid ist.

7. Mittel zur Verwendung in der Therapie und/oder der Prävention von Nierenkrankheiten gemäß irgendeinem der Ansprüche 1 bis 6, worin das Nukleinsäuremolekül, welches das Antisense-Oligonukleotid gegen Casein Kinase 2 bildet, eine Sequenz wie in irgendeiner der in SEQ ID NOs: 1-10 gezeigten Sequenzen hat.

8. Mittel zur Verwendung in der Therapie und/oder der Prävention von Nierenkrankheiten gemäß irgendeinem der Ansprüche 1 bis 7, worin das Antisense-Oligonukleotid gegen Casein Kinase 2 eine Sequenz hat wie in SEQ ID NO: 1 gezeigt.

9. Mittel zur Verwendung in der Therapie und/oder der Prävention von Nierenkrankheiten gemäß irgendeinem der Ansprüche 1 bis 8, wobei die Nierenkrankheit glomeruläre Nephritis, interstitielle Nephritis, Nephrosklerose, diabetische Nephropathie oder chronisches oder akutes Nierenversagen ist.

10. Mittel zur Verwendung in der Therapie und/oder der Prävention von Nierenkrankheiten gemäß irgendeinem der Ansprüche 1 bis 9, wobei die Nierenkrankheit eine Nephritis ist, die nicht durch Diabetes verursacht wurde, oder die Nierenkrankheit durch eine Erhöhung der Expression von Casein Kinase 2 oder Erhöhung der Enzymaktivität von Casein Kinase 2 **gekennzeichnet** ist.

11. Verfahren zur Diagnose von Nierenkrankheiten, das die Messung der Aktivität und/oder des Gehalts von Casein Kinase 2 und/oder die Messung der Expressionsmenge von Casein Kinase 2-Gen in einer Probe, die vom Körper getrennt wurde, umfasst.

12. Verfahren gemäß Anspruch 11, worin die Probe Zellen sind, aus denen die Niere aufgebaut ist.

13. Verfahren gemäß Anspruch 11 oder 12, worin die Casein Kinase 2 die α-Untereinheit und/oder die α'-Untereinheit ist.

14. Verfahren gemäß irgendeinem der Ansprüche 11 bis 13, worin die Nierenkrankheit glomeruläre Nephritis, interstitielle Nephritis, Nephrosklerose, diabetische Nephropathie oder chronisches oder akutes Nierenversagen ist.

15. Verfahren gemäß irgendeinem der Ansprüche 11 bis 13, worin die Nierenkrankheit eine Nephritis ist, die nicht durch Diabetes verursacht wurde, oder die Nierenkrankheit durch eine Erhöhung der Expression von Casein Kinase 2 oder Erhöhung der Enzymaktivität von Casein Kinase 2 **gekennzeichnet** ist.

16. Verfahren gemäß irgendeinem der Ansprüche 11 bis 15, welches die Messung der Expressionsmenge von Casein Kinase 2-Gen umfasst.

17. Ein Oligonukleotid mit einer Nukleotidsequenz, die komplementär zu einem Bereich in einer Sense-Kette oder Antisense-Kette des Casein Kinase 2-Gens ist, welches für das Verfahren gemäß irgendeinem der Ansprüche 11 bis 16 verwendet wird.

18. Oligonukleotid gemäß Anspruch 17, das ein Primer für Genamplifikation oder eine Sonde für Gendetektion ist.

## Revendications

1. Agent destiné à une utilisation en thérapie et/ou prévention des maladies rénales, comprenant comme principe actif une molécule d'acide nucléique qui inhibe l'expression de la caséine kinase 2.

2. Agent destiné à une utilisation en thérapie et/ou prévention des maladies rénales selon la revendication 1, dans lequel ladite caséine kinase 2 est originaire de cellules constituant le rein.

3. Agent destiné à une utilisation en thérapie et/ou prévention des maladies rénales selon la revendication 1 ou 2, dans lequel ladite molécule d'acide nucléique est une molécule d'acide nucléique qui inhibe l'expression de la sous-unité α et/ou la sous-unité α' de la caséine kinase 2.

4. Agent destiné à une utilisation en thérapie et/ou prévention des maladies rénales selon l'une quelconque des revendications 1 à 3, dans lequel ladite molécule d'acide nucléique qui inhibe l'expression de la caséine kinase 2 est un oligonucléotide antisens qui cible l'ARNm codant pour la caséine kinase 2, et qui peut inhiber l'expression de la caséine kinase 2

5. Agent destiné à une utilisation en thérapie et/ou prévention des maladies rénales selon l'une quelconque des revendications 1 à 4, dans lequel ledit oligonucléotide antisens possède 12 à 50 bases ayant une séquence choisie dans le groupe constitué par les régions codantes, les régions non traduite en 3', la région non traduite en 5', la coiffe en 5' et les jonctions intron/exon de l'ARNm codant pour la caséine kinase 2.

6. Agent destiné à une utilisation en thérapie et/ou prévention des maladies rénales selon l'une quelconque des revendications 1 à 5, dans lequel ladite molécule d'acide nucléique dudit oligonucléotide antisens contre la caséine kinase 2 est un S-oligonucléotide.

7. Agent destiné à une utilisation en thérapie et/ou prévention des maladies rénales selon l'une quelconque des revendications 1 à 6, dans lequel ladite molécule d'acide nucléique formant ledit oligonucléotide antisens contre la caséine kinase 2 a une séquence présentée dans l'une quelconque de SEQ ID NOs: 1 à 10.

8. Agent destiné à une utilisation en thérapie et/ou prévention des maladies rénales selon l'une quelconque des revendications 1 à 7, dans lequel ledit oligonucléotide antisens contre la caséine kinase 2 a une séquence présentée dans SEQ ID NO: 1.

9. Agent destiné à une utilisation en thérapie et/ou prévention des maladies rénales selon l'une quelconque des revendications 1 à 8, dans lequel ladite maladie rénale est une néphrite glomérulaire, une néphrite interstitielle, une néphrosclérose, une néphropathie diabétique ou une insuffisance rénale aiguë ou chronique.

10. Agent destiné à une utilisation en thérapie et/ou prévention des maladies rénales selon l'une quelconque des revendications 1 à 9, dans lequel ladite maladie rénale est une néphrite autre que celle résultant d'un diabète, ou ladite maladie rénale est **caractérisée par** une augmentation de l'expression de la caséine kinase 2 ou une augmentation de l'activité enzymatique de la caséine kinase 2.

11. Procédé pour le diagnostic des maladies rénales, comprenant la mesure de l'activité et/ou la teneur de la caséine kinase 2, et/ou la mesure de la quantité d'expression du gène de la caséine kinase 2 dans un échantillon séparé du corps.

12. Procédé selon la revendication 11, dans lequel ledit échantillon est des cellules constituant le rein.

13. Procédé selon la revendication 11 ou 12, dans lequel ladite caséine kinase 2 est une sous-unité α et/ou une sous-unité α'.

14. Procédé selon l'une quelconque des revendications 11 à 13, dans lequel ladite maladie rénale est une néphrite glomérulaire, une néphrite interstitielle, une néphrosclérose, une néphropathie diabétique ou une insuffisance rénale aiguë ou chronique.

15. Procédé selon l'une quelconque des revendications 11 à 13, dans lequel ladite maladie rénale est une néphrite autre que celle résultant d'un diabète, ou ladite maladie rénale est **caractérisée par** une augmentation de l'expression de la caséine kinase 2 ou une augmentation de l'activité enzymatique de la caséine kinase 2.

16. Procédé selon l'une quelconque des revendications 11 à 15, comprenant la mesure de la quantité d'expression du gène de la caséine kinase 2.

17. Oligonucléotide ayant une séquence nucléotidique complémentaire d'une région dans la chaîne sens ou la chaîne antisens du gène de la caséine kinase 2, qui est utilisé pour le procédé selon l'une quelconque des revendications 11 à 16.

18. Oligonucléotide selon la revendication 17, qui est une amorce pour l'amplification d'un gène ou une sonde pour la détection d'un gène.


### SEQUENCE LISTING

<110> TORAY INDUSTRIES. INC.
<120> Agent for therapy and/or prevention of kidney diseases and method for diagnosing kidney diseases
<130> 02796
<160> 27

<210> 1
<211> 21
<212> DNA
<213> Artificial Sequence
<220>
<223> antisense oligonucleotide used for inhibition of casein kinase 2 α subunit gene
<400> 1
   gtaatcatct tgattacccc a 21

<210> 2
<211> 21
<212> DNA
<213> Artificial Sequence
<220>
<223> antisense oligonucleotide used for inhibition of casein kinase 2 β subunit gene
<400> 2
   ggttggccgg ccgcttgggc c 21

<210> 3
<211> 20
<212> DNA
<213> Artificial Sequence
<220>
<223> antisense oligonucleotide used for inhibition of casein kinase 2 α' subunit gene
<400> 3
   ttcaaatacc aaagctggtg 20

<210> 4
<211> 20
<212> DNA
<213> Artificial Sequence
<220>
<223> antisense oligonucleotide used for inhibition of casein kinase 2 α' subunit gene
<400> 4
   atcaaagtct gtcaggatct 20

<210> 5
<211> 20
<212> DNA
<213> Artificial Sequence
<220>
<223> antisense oligonucleotide used for inhibition of casein kinase 2 α' subunit gene
<400> 5
   tggataaagt tttcccagcg 20

<210> 6
<211> 21
<212> DNA
<213> Artificial Sequence
<220>
<223> antisense oligonucleotide used for inhibition of casein kinase 2 α' subunit gene
<400> 6
   accaagtttt cgaacccagt t 21

<210> 7
<211> 20
<212> DNA
<213> Artificial Sequence
<220>
<223> antisense oligonucleotide used for inhibition of casein kinase 2 β subunit gene
<400> 7
   ctgctcatct tgacgtcagc 20

<210> 8
<211> 20
<212> DNA
<213> Artificial Sequence
<220>
<223> antisense oligonucleotide used for inhibition of casein kinase 2 β subunit gene
<400> 8
   ctcagagcta aagcctcgtg 20

<210> 9
<211> 20
<212> DNA
<213> Artificial Sequence
<220>
<223> antisense oligonucleotide used for inhibition of casein kinase 2 β subunit gene
<400> 9
   acccgaccgc ggcaggcgaa 20

<210> 10
<211> 20
<212> DNA
<213> Artificial Sequence
<220>
<223> antisense oligonucleotide used for inhibition of casein kinase 2 β subunit gene
<400> 10
   gcggcgaccg ctacagcgca 20

<210> 11
<211> 20
<212> DNA
<213> Artificial Sequence
<220>
<223> oligonucleotide primer used for PCR for amplification of rat case in kinase 2 β subunit gene
<400> 11
   ccgcggacat aaagatgagt 20

<210> 12
<211> 20
<212> DNA
<213> Artificial Sequence
<220>
<223> oligonucleotide primer used for PCR for amplification of rat case in kinase 2 β subunit gene
<400> 12
   aaaccagtgc cgaagtatgc 20

<210> 13
<211> 20
<212> DNA
<213> Artificial Sequence
<220>
<223> oligonucleotide primer used for PCR for amplification of rat case in kinase 2 a subunit gene
<400> 13
   agaaagcttc ggctastaga 20

<210> 14
<211> 20
<212> DNA
<213> Artificial Sequence
<220>
<223> oligonucleotide primer used for PCR for amplification of rat case in kinase 2 α subunit gene
<400> 14
   actgaagaaa tccctgacat 20

<210> 15
<211> 2187
<212> DNA
<213> homo sapiens
<400> 15
<210> 16
<211> 1677
<212> DNA
<213> homo sapiens
<400> 16
<210> 17
<211> 648
<212> DNA
<213> homo sapiens
<400> 17
   atg agc agc tca gag gag gtg tcc tgg att tcc tgg ttc tgt ggg ctc 48
   Met Ser Ser Ser Glu Glu Val Ser Trp Ile Ser Trp Phe Cys Gly Leu
   1 5 10 15
   cgt ggc aat gaa ttc ttc tgt gaa gtg gat gaa gac tac atc cag gac 96
   Arg Gly Asn Glu Phe Phe Cys Glu Val Asp Glu Asp Tyr Ile Gln Asp
   20 25 30
   aaa ttt aat ctt act gga ctc aat gag cag gtc cct cac tat cga caa 144
   Lys Phe Asn Leu Thr Gly Leu Asn Glu Gln Val Pro His Tyr Arg Gln
   35 40 45
   gct cta gac atg atc ttg gac ctg gag cct gat gaa gaa ctg gaa gac 192
   Ala Leu Asp Met Ile Leu Asp Leu Glu Pro Asp Glu Glu Leu Glu Asp
   50 55 60
   aac ccc aac cag agt gac ctg att gag cag gca gcc gag atg ctt tat 240
   Asn Pro Asn Gln Ser Asp Leu Ile Glu Gln Ala Ala Glu Met Leu Tyr
   65 70 75 80
   gga ttg atc cac gcc cgc tac atc ctt acc aac cgt ggc atc gcc cag 288
   Gly Leu Ile His Ala Arg Tyr Ile Leu Thr Asn Arg Gly Ile Ala Gln
   85 90 95
   atg ttg gaa aag tac cag caa gga gac ttt ggt tac tgt cct cgt gtg 336
   Met Leu Glu Lys Tyr Gln Gln Gly Asp Phe Gly Tyr Cys Pro Arg Val
   100 105 110
   tac tgt gag aac cag cca atg ctt ccc att ggc ctt tca gac atc cca 384
   Tyr Cys Glu Asn Gln Pro Met Leu Pro Ile Gly Leu Ser Asp Ile Pro
   115 120 125
   ggt gaa gcc atg gtg aag ctc tac tgc ccc aag tgc atg gat gtg tac 432
   Gly Glu Ala Met Val Lys Leu Tyr Cys Pro Lys Cys Met Asp Val Tyr
   130 135 140
   aca ccc aag tca tca aga cac cat cac acg gat ggc gcc tac ttc ggc 480
   Thr Pro Lys Ser Ser Arg His His His Thr Asp Gly Ala Tyr Phe Gly
   145 150 155 160
   act ggt ttc cct cac atg ctc ttc atg gtg cat ccc gag tac cgg ccc 528
   Thr Gly Phe Pro His Met Leu Phe Met Val His Pro Glu Tyr Arg Pro
   165 170 175
   aag aga cct gcc aac cag ttt gtg ccc agg ctc tac ggt ttc aag atc 576
   Lys Arg Pro Ala Asn Gln Phe Val Pro Arg Leu Tyr Gly Phe Lys Ile
   180 185 190
   cat ccg atg gcc tac cag ctg cag ctc caa gcc gcc agc aac ttc aag 624
   His Pro Met Ala Tyr Gln Leu Gln Leu Gln Ala Ala Ser Asn Phe Lys
   195 200 205
   agc cca gtc aag acg att cgc tga 648
   Ser Pro Val Lys Thr Ile Arg
   210 215

<210> 18
<211> 2180
<212> DNA
<213> rat
<400> 18
   gggttcttcc ttcatatgcc agctagagag ccttgcctcc ctggagctca tagtccaggc 60
   tgtgttctgg ggacctgagg ggtgtggtca gggcacagag actctctcag agcactggga 120
   gtgggattcc tgctgtaagg gaagggatgt cataggtcag ttgaccaatg accagaccta 180
   aacatggctc ccagccttag gcatgcggag cctaggagtc ccttcaaccc tggcctgtga 240
   tttttccagc tcagatgaca aagacatcta ggcccagtgt cttgagaatc cctatagtca 300
   aggattagag tcctcctcag tgagtcagct cccccagtca gtaccctcac aagatatcaa 360
   ccgaattcgg tttttttttt tttttttttt taaatatgta aggctttcaa tttattacag 420
   atcacccaag aacataatga tatacatgta gtcagaaaac acgatgtaga aatcatagtg    480
   agttgtccag acatagtcag tagattattc tttctggcat gctccagtgt caagacctca    540
   ataaagagca ctaaaatcct tccatacaat taagtatcag cgatgtacca tattgcagaa    600
   aggggtggct gagcaacagt ttgttgatac tataagactt ccatgctcaa cagtcgtagc    660
   gttgcactca gcacagctct ggtttccata tacaaatttt ccatctcgta ggggagcgcg    720
   gctagtgccg ctgccgcttc caccgcagta actgccagat cttccaacat cacgttcagc    780
   tttgtccgtc aacctgtctg ac atg tcg gga ccc gtg cca agc agg gcc aga    832
                            Met Ser Gly Pro Val Pro Ser Arg Ala Arg
                            1               5                   10
   gtt tac aca gat gtt aac aca cac aga ccc cga gag tac tgg gac tat    880
   Val Tyr Thr Asp Val Asn Thr His Arg Pro Arg Glu Tyr Trp Asp Tyr
                   15                  20                  25
   gaa tca cat gtg gtg gaa tgg gga aat caa gat gac tac cag ctt gtt    928
   Glu Ser His Val Val Glu Trp Gly Asn Gln Asp Asp Tyr Gln Leu Val
               30                  35                  40
   cga aaa tta ggc agg ggc aaa tac agt gaa gtg ttt gag gcc atc aat    976
   Arg Lys Leu Gly Arg Gly Lys Tyr Ser Glu Val Phe Glu Ala Ile Asn
           45                  50                  55
   atc aca aat aat gaa aaa gtt gtt gtt aaa att ctc aag cca gta aaa    1024
   Ile Thr Asn Asn Glu Lys Val Val Val Lys Ile Leu Lys Pro Val Lys
       60                  65                  70
   aag aag aaa att aag cgt gaa ata aag att ttg gag aat tta aga ggt    1072
   Lys Lys Lys Ile Lys Arg Glu Ile Lys Ile Leu Glu Asn Leu Arg Gly
   75                  80                  85                  90
   ggg ccc aac atc atc aca ctt gca gac att gtg aaa gac cct gtg tct    1120
   Gly Pro Asn Ile Ile Thr Leu Ala Asp Ile Val Lys Asp Pro Val Ser
                   95                  100                 105
   cga acc cct gcc ttg gtt ttt gaa cat gta aac aac aca gac ttc aag    1168
   Arg Thr Pro Ala Leu Val Phe Glu His Val Asn Asn Thr Asp Phe Lys
   110 115 120
   caa ttg tac cag acg tta aca gac tat gac att cga ttt tac atg tat 1216
   Gln Leu Tyr Gln Thr Leu Thr Asp Tyr Asp Ile Arg Phe Tyr Met Tyr
   125 130 135
   gaa att ctg aaa gcc ctg gat tat tgt cac agc atg ggg att atg cac 1264
   Glu Ile Leu Lys Ala Leu Asp Tyr Cys His Ser Met Gly Ile Met His
   140 145 150
   aga gac gtg aaa ccg cat aat gtc atg att gat cat gag cac aga aag 1312
   Arg Asp Val Lys Pro His Asn Val Met Ile Asp His Glu His Arg Lys
   155 160 165 170
   ctt cgg cta ata gat tgg ggt tta gca gag ttt tac cat cct ggc caa 1360
   Leu Arg Leu Ile Asp Trp Gly Leu Ala Glu Phe Tyr His Pro Gly Gln
   175 180 185
   gag tat aat gtc cga gtt gct tcc cga tat ttc aaa ggt cca gag cta 1408
   Glu Tyr Asn Val Arg Val Ala Ser Arg Tyr Phe Lys Gly Pro Glu Leu
   190 195 200
   ctt gta gat tat cag atg tac gat tat agt ttg gat atg tgg agc ttg 1456
   Leu Val Asp Tyr Gln Met Tyr Asp Tyr Ser Leu Asp Met Trp Ser Leu
   205 210 215
   ggt tgt atg ctg gca agt atg atc ttc cgg aag gag cca ttt ttc cat 1504
   Gly Cys Met Leu Ala Ser Met Ile Phe Arg Lys Glu Pro Phe Phe His
   220 225 230
   gga cat gac aat tat gat cag ttg gtg agg ata gcc aag gtt ctg gga 1552
   Gly His Asp Asn Tyr Asp Gln Leu Val Arg Ile Ala Lys Val Leu Gly
   235 240 245 250
   acg gaa gat tta tat gac tat att gac aag tac aac att gaa tta gat 1600
   Thr Glu Asp Leu Tyr Asp Tyr Ile Asp Lys Tyr Asn Ile Glu Leu Asp
   255 260 265
   cca cgt ttc aac gat atc ttg ggc aga cac tcc cgt aag cga tgg gaa 1648
   Pro Arg Phe Asn Asp Ile Leu Gly Arg His Ser Arg Lys Arg Trp Glu
   270 275 280
   cgc ttt gtc cac agt gaa aac cag cac ctt gtc agc ccc gag gcc ttg 1696
   Arg Phe Val His Ser Glu Asn Gln His Leu Val Ser Pro Glu Ala Leu
   285 290 295
   gat ttt ctg gac aag ctg ctg cga tac gac cac cag tct cgg ctc act 1744
   Asp Phe Leu Asp Lys Leu Leu Arg Tyr Asp His Gln Ser Arg Leu Thr
   300 305 310
   gca aga gag gcc atg gag cac cct tac ttc tac act gtc gtg aag gac 1792
   Ala Arg Glu Ala Met Glu His Pro Tyr Phe Tyr Thr Val Val Lys Asp
   315 320 325 330
   cag gct cga atg agt tcg gct ggc atg gca ggg ggc agc aca cct gtc 1840
   Gln Ala Arg Met Ser Ser Ala Gly Met Ala Gly Gly Ser Thr Pro Val
   335 340 345
   agt agc gcc aat atg atg tca ggg att tct tca gtg cca acc cct tca 1888
   Ser Ser Ala Asn Met Met Ser Gly Ile Ser Ser Val Pro Thr Pro Ser
   350 355 360
   ccc ctg gga cct ctg gca ggc tca ccc gtg att gct gct gcc aac tca 1936
   Pro Leu Gly Pro Leu Ala Gly Ser Pro Val Ile Ala Ala Ala Asn Ser
   365 370 375
   ctt ggg ata ccc gta cca gct gcc gct ggc gct cag cag taa tgaccccat 1987
   Leu Gly Ile Pro Val Pro Ala Ala Ala Gly Ala Gln Gln
   380 385 390
   ctgtcttctg atgcctgggc agaggtggga cgtccaccct ctccttaatg cagcttgcgc 2047
   ctggttggga ggggtgagaa cacttcagaa gcaccgtgtc tgaaccgttg cttgtggatt 2107
   tagtagttga gtcataaaaa aaattatagg ctgattttct ttcttttttt tttttttttt 2167
   tttaaaaaaa ccg 2180

<210> 19
<211> 1964
<212> DNA
<213> rat
<400> 19
   ggctgccgcg gcccggtcgg ctttctgcgc tgtagcggtc tctgccgttc cttggaagca    60
   cagctcccct tccccgcccc agtcccagtc cccgtccggc cgcggacata aag atg       116
                                                              Met
                                                              1
   agt agc tct gag gag gtg tcc tgg att tcc tgg ttc tgt ggg ctc cgt      164
   Ser Ser Ser Glu Glu Val Ser Trp Ile Ser Trp Phe Cys Gly Leu Arg
               5                   10                  15
   ggt aat gaa ttc ttc tgt gag gtg gat gaa gac tac atc cag gac aaa      212
   Gly Asn Glu Phe Phe Cys Glu Val Asp Glu Asp Tyr Ile Gln Asp Lys
           20                  25                  30
   ttt aat ctt act gga ctc aat gag cag gtg cct cac tat cga caa gcc      260
   Phe Asn Leu Thr Gly Leu Asn Glu Gln Val Pro His Tyr Arg Gln Ala
       35                  40                  45
   cta gac atg atc ttg gac ctg gaa cct gat gaa gag ctg gaa gac aac      308
   Leu Asp Met Ile Leu Asp Leu Glu Pro Asp Glu Glu Leu Glu Asp Asn
   50                  55                  60                  65
   ccc aac cag agt gac ttg att gag cag gcg gcc gag atg ctc tat ggg      356
   Pro Asn Gln Ser Asp Leu Ile Glu Gln Ala Ala Glu Met Leu Tyr Gly
                   70                  75                  80
   ttg atc cac gcc cgc tac atc ctc acc aac cgg ggc att gca caa atg      404
   Leu Ile His Ala Arg Tyr Ile Leu Thr Asn Arg Gly Ile Ala Gln Met
   85 90 95
   ttg gaa aag tac cag caa gga gac ttt ggc tac tgt cct cga gta tac 452
   Leu Glu Lys Tyr Gln Gln Gly Asp Phe Gly Tyr Cys Pro Arg Val Tyr
   100 105 110
   tgt gag aac cag ccg atg ctt ccc atc ggc ctt tcg gac atc cca gga 500
   Cys Glu Asn Gln Pro Met Leu Pro Ile Gly Leu Ser Asp Ile Pro Gly
   115 120 125
   gag gcc atg gtg aag ctc tac tgc ccc aag tgc atg gac gtg tac aca 548
   Glu Ala Met Val Lys Leu Tyr Cys Pro Lys Cys Met Asp Val Tyr Thr
   130 135 140 145
   ccc aag tcc tct agg cac cac cac acg gat ggc gca tac ttc ggc act 596
   Pro Lys Ser Ser Arg His His His Thr Asp Gly Ala Tyr Phe Gly Thr
   150 155 160
   ggt ttc cct cac atg ctc ttc atg gtg cat ccc gag tac cgg ccc aag 644
   Gly Phe Pro His Met Leu Phe Met Val His Pro Glu Tyr Arg Pro Lys
   165 170 175
   cgg ccg gcc aac cag ttt gtg ccc agg ctc tac ggt ttc aag atc cat 692
   Arg Pro Ala Asn Gln Phe Val Pro Arg Leu Tyr Gly Phe Lys Ile His
   180 185 190
   cca atg gcc tac cag ctg cag ctc caa gcc gcc agc aac ttc aag agc 740
   Pro Met Ala Tyr Gln Leu Gln Leu Gln Ala Ala Ser Asn Phe Lys Ser
   195 200 205
   cca gtc aag acg att cgc tga gtgccctccc acctcctctg cctgtgacac cacc 795
   Pro Val Lys Thr Ile Arg
   210
   gtccctccgc tgccaccctt tcaggaagtc tatggttttt agtttaaatt aaaggaattg 855
   ttactgtggt ggggatatga aataaaggaa gagaaggcta aaaaaaaaaa aaaaaaccga 915
   attcggcatc tcacctaggt gtatacagct gcacattacc aatggctgca gaacacccaa 975
   tcatgacctc tagtgcttta tagcaaaagt gtaaagtggt acctgggtga ccagcattgc 1035
   catggttaca tccatttgcg taatcaagca gctgtgcaac tgctcttcta acagatgaac 1095
   agctgcatgc atctccagca ggcatggatt gtctcttgtc tatgatcctc agtgcgttca 1155
   cttagagtat ttctagagtt tgagtccttg ccgtgataga gccatgtagg gaatgcactg 1215
   attgcatgtt accccaagcg tcatgaaacc ttccgacacg gtgacctatt taatggtctt 1275
   gtttgttgac atgacaaatt aacattctta gagttacatc tcgaaaaaag catttgtgat 1335
   agataagccc tttgagcctt gtggctaaat ttttgtggct ttgtttaact ttcaaaggtt 1395
   atatatgcac taaccttttt tgatggctaa gtaggcttta aattacagta aattttttca 1455
   aataaaactg gctgtaaaat atattttgaa ttagagttgt tcactttttc atagctactt 1515
   atgttttttt cccaataatt tatttcacat ctctaccagt gaacgcagcc catcactaga 1575
   aaatgacctt gtcctgattt cagtttcaac tattagtttt aaagctcact gttgaataag 1635
   aggaagtggt ggtgcatttt aaattgactt tcatgtgctt ttaaaatatg acagatctcc 1695
   ttgataatgt acttttattt gatctcaagt tgtataaaac caataaattt gtgttacttt 1755
   gattgcagta gtatcttatg catagtgatt ccatgttata tgcagactag ttaggcaact 1815
   gttttcttag ttacaagctt cacttttgtg cagttaaaaa aacaaaagta ggctacagtc 1875
   tgtgccatgt tgatgtacag tttctgaaat tgttttacaa gactttgata ataaaaccct 1935
   taaactcaca aaaaaaaaaa aaaaaaacc 1964

<210> 20
<211> 21
<212> DNA
<213> Artificial Sequence
<220>
<223> siRNA for inhibiting human kasein kinase 2 beta subunit RNA
<400> 20
   cuaccgacaa gcucuagact t 21

<210> 21
<211> 21
<212> RNA
<213> Artificial Sequence
<220>
<223> siRNA for inhibiting human kasein kinase 2 beta subunit RNA
<400> 21
   gucuagagcu ugucgguagt t 21

<210> 22
<211> 21
<212> RNA
<213> Artificial Sequence
<220>
<223> siRNA for inhibiting human kasein kinase 2 beta subunit RNA
<400> 22
   cuaccgacaa gcucuagaca t 21

<210> 23
<211> 21
<212> RNA
<213> Artificial Sequence
<220>
<223> siRNA for inhibiting human kasein kinase 2 beta subunit RNA
<400> 23
   gucuagagcu ugucgguagt g 21

<210> 24
<211> 21
<212> RNA
<213> Artificial Sequence
<220>
<223> siRNA for inhibiting rat kasein kinase 2 beta subunit RNA
<400> 24
   aucuuacugg acucaaugat t 21

<210> 25
<211> 21
<212> RNA
<213> Artificial Sequence
<220>
<223> siRNA for inhibiting rat kasein kinase 2 beta subunit RNA
<400> 25
   gauggcuguu cgagaucugt t 21

<210> 26
<211> 10
<212> PRT
<213> Artificial Sequence
<220>
<223> substrate of kasein kinase 2 used for determination of kasein kin ase 2 activity
<400> 26
   Arg Arg Arg Glu Glu Glu Thr Glu Glu Glu
   1 5 10

<210> 27
<211> 9
<212> PRT
<213> Artificial Sequence
<220>
<223> substrate of kasein kinase 2 used for determination of kasein kin ase 2 activity
<400> 27
   Arg Arg Glu Glu Glu Thr Glu Glu Glu
   1 5